(19) Europäisches Patentamt
European Patent Office
Office européen des brevets

(11) Veröffentlichungsnummer: **0 003 539**
**B1**

(12) **EUROPÄISCHE PATENTSCHRIFT**

(45) Veröffentlichungstag der Patentschrift: **08.04.81**

(51) Int. Cl.³: **C 07 D 271/10,** C 07 D 285/12, C 07 D 249/08, C 07 D 249/10, C 07 D 249/12, A 01 N 43/82 // C07C159/00, C07C109/087

(21) Anmeldenummer: **79100240.5**

(22) Anmeldetag: **29.01.79**

(54) **N-Azolylalkyl-halogenacetanilide, Verfahren zu ihrer Herstellung sowie ihre Verwendung als Herbizide.**

(30) Priorität: **10.02.78 DE 2805757**

(43) Veröffentlichungstag der Anmeldung: **22.08.79 Patentblatt 79/17**

(45) Bekanntmachung des Hinweises auf die Patenterteilung: **08.04.81 Patentblatt 81/14**

(84) Benannte Vertragsstaaten: **BE CH DE FR GB IT NL SE**

(56) Entgegenhaltungen: **DE-A-2 625 285**
**GB-A-1 307 283**

(73) Patentinhaber: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal 1 (DE)**
Erfinder: **Draber, Wilfried, Dr., In den Birken 81, D-5600 Wuppertal 1 (DE)**
Erfinder: **Thomas, Rudolf, Dr., Wilkhausstrasse 129, D-5600 Wuppertal 2 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**
Erfinder: **Lunkenheimer, Winfried, Dr., Bismarckstrasse 29, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



BUNDESDRUCKEREI BERLIN

N-Azolylalkyl-halogenacetanilide,
Verfahren zu ihrer Herstellung sowie ihre Verwendung als Herbizide

Die vorliegende Erfindung betrifft neue N-Azolylalkylhalogenacetanilide, ein Verfahren zu ihrer Herstellung sowie ihre Verwendung als Herbizide.

Es ist bereits bekannt geworden, daß man 2,6-Diäthyl-N-methyoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag [1977]). Diese Verbindung ist jedoch, vor allem gegenüber Dicotylen nicht immer ausreichend wirksam und in ihrer Selektivität nicht immer ganz befriedigend.

Es wurden nun als neue Verbindungen die N-Azolylalkylhalogenacetanilide der Formel

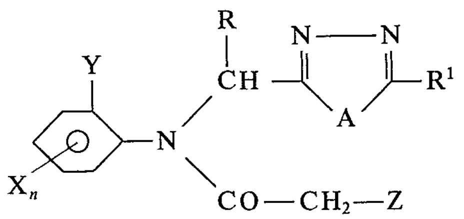

(I)

in welcher

A für Sauerstoff, Schwefel oder die Gruppierung $>NR^2$ steht,
R für Wasserstoff oder Methyl steht,
$R^1$ für Wasserstoff, Alkyl mit 1 bis 4 Kohlenstoffatomen, Halogenalkyl mit bis zu 3 Kohlenstoffatomen und bis zu 5 gleichen oder verschiedenen Halogenatomen, Alkenyl mit 2 bis 4 Kohlenstoffatomen, Alkinyl mit 2 bis 4 Kohlenstoffatomen, Cycloalkyl mit 5 bis 7 Kohlenstoffatomen, Halogen, gegebenenfalls durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen substituiertes Aryl mit 6 bis 10 Kohlenstoffatomen sowie für Aralkyl mit 6 bis 10 Kohlenstoffatomen im Arylteil und 1 bis 4 Kohlenstoffatomen im Alkylteil steht, wobei der Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, oder $R^1$ für die Gruppierungen $-OR^3$, $-SR^3$ und $-NR^2R^3$ steht,
$R^2$ für Wasserstoff, Alkyl mit 1 bis 4 Kohlenstoffatomen oder für gegebenenfalls durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen substituiertes Aryl mit 6 bis 10 Kohlenstoffatomen steht,
$R^3$ für Wasserstoff, Alkyl mit 1 bis 4 Kohlenstoffatomen, Halogenalkyl mit bis zu 3 Kohlenstoffatomen und bis zu 5 gleichen oder verschiedenen Halogenatomen, Alkenyl mit 2 bis 4 Kohlenstoffatomen, Alkinyl mit 2 bis 4 Kohlenstoffatomen, Cycloalkyl mit 5 bis 7 Kohlenstoffatomen oder für Aralkyl mit 6 bis 10 Kohlenstoffatomen im Arylteil und 1 bis 4 Kohlenstoffatomen im Alkylteil steht, wobei der Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen,
X für Alkyl mit 1 bis 4 Kohlenstoffatomen steht,
Y für Alkyl mit 1 bis 4 Kohlenstoffatomen oder Halogen steht,
Z für Halogen steht und
n für die Zahlen 0, 1 oder 2 steht,

gefunden.

Weiterhin wurde gefunden, daß man die N-Azolylalkyl-halogenacetanilide der Formel (I) erhält, wenn man N-Azolylalkylaniline der Formel

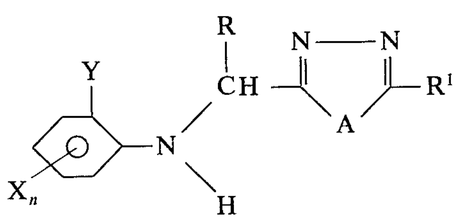

(II)

in welcher
A, R, $R^1$, X, Y und n die oben angegebene Bedeutung haben,

mit Halogenessigsäurechloriden bzw. -anhydriden der Formeln

$$Z—CH_2—CO—Cl \qquad (IIIa)$$

bzw.

$$(Z—CH_2—CO)_2O \qquad (IIIb)$$

in welchen
Z die oben angegebene Bedeutung hat,

in Gegenwart eines Verdünnungsmittels und gegebenenfalls in Gegenwart eines Säurebindemittels umsetzt.

Außerdem wurde gefunden, daß die N-Azolylalkylhalogenacetanilide der Formel (I) starke herbizide, insbesondere auch selektiv-herbizide Eigenschaften aufweisen.

Überraschenderweise sind die erfindungsgemäßen N-Azolylalkyl-halogenacetanilide dem bekannten 2,6-Diäthyl-N-methoxymethyl-chloracetanilid bei gleichwertiger Wirkung gegen Monocotyle in ihrer Wirkung gegen Dicotyle weitgehend überlegen. Vor allem aber zeigen sie überraschenderweise eine bessere Selektivität in wichtigen Kulturpflanzen als die zuvor erwähnte, vorbekannte Verbindung, welche ein hochaktiver Wirkstoff gleicher Wirkungsart ist. Die erfindungsgemäßen Stoffe stellen somit eine wertvolle Bereicherung der Technik dar.

Die erfindungsgemäßen N-Azolylalkyl-halogenacetanilide sind durch die Formel (I) allgemein definiert. In der Formel (I) steht A vorzugsweise für Sauerstoff, Schwefel oder die Gruppierung > $NR^2$, worin $R^2$ vorzugsweise für Wasserstoff, geradkettiges oder verzweigtes Alkyl mit 1 bis 4 Kohlenstoffatomen sowie für Phenyl steht, wobei dieser Phenylrest substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbeosndere Fluor und Chlor genannt seien. $R^1$ steht in der Formel (I) vorzugsweise für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Fluor, Chlor oder Brom. $R^1$ steht ferner vorzugsweise für Phenyl, wobei dieser Phenylrest substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^1$ steht ferner vorzugsweise für Benzyl, wobei dieser Benzylrest im Phenylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Außerdem steht $R^1$ für die Gruppierungen $-OR^3$, $-SR^3$ und $-NR^2R^3$, worin $R^2$ vorzugsweise für diejenigen Reste steht, die oben bereits vorzugsweise für diesen Rest genannt wurden, $R^3$ steht in diesen Gruppierungen vorzugsweise für Wasserstoff, gradkettiges oder verzweigtes Alkyl mit 1 bis 4 Kohlenstoffatomen, Halogenalkyl mit 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Benzyl, wobei dieser Benzylrest im Phenylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei.

In der Formel (I) steht X vorzugsweise für gradkettiges oder verzweigtes Alkyl mit 1 bis 4 Kohlenstoffatomen. Y steht in der Formel (I) vorzugsweise für gradkettiges oder verzweigtes Alkyl mit 1 bis 4 Kohlenstoffatomen oder für Fluor, Chlor und Brom. Z steht vorzugsweise für Chlor, Brom und Jod; der Index n steht für die Zahlen 0, 1 oder 2.

Als Beispiel für erfindungsgemäße N-Azolylalkylhalogenacetanilide der Formel (I) seien im einzelnen genannt:

2,6-Diäthyl-N-[(5-methyl-1,3,4-oxadiazol-2-yl-methyl)]-chloracetanilid
2-Äthyl-6-methyl-N-[(5-methyl-1,3,4-oxadiazol-2-yl-methyl)]-chloracetanilid
2,6-Dimethyl-N-[(5-methyl-1,3,4-oxadiazol-2-yl-methyl)]-chloracetanilid
2-tert.-Butyl-N-[(5-methyl-1,3,4-oxadiazol-2-yl-methyl)]-chloracetanilid
2-Chlor-6-tert.-butyl-N-[(5-methyl-1,3,4-oxadiazol-2-yl-methyl)]-chloracetanilid
2-Chlor-3-methyl-N-[(5-methyl-1,3,4-oxadiazol-2-yl-methyl)]-chloracetanilid
2-Chlor-6-methyl-N-[(5-methyl-1,3,4-oxadiazol-2-yl-methyl)]-chloracetanilid
2-Chlor-6-isopropyl-N-[(5-methyl-1,3,4-oxadiazol-2-yl-methyl)]-chloracetanilid
2-Isopropyl-N-[(5-methyl-1,3,4-oxadiazol-2-yl-methyl)]-chloracetanilid
2-Äthyl-6-isopropyl-N-[(5-methyl-1,3,4-oxadiazol-2-yl-methyl)]-chloracetanilid
2-Brom-6-tert.-butyl-N-[(5-methyl-1,3,4-oxadiazol-2-yl-methyl)]-chloracetanilid
2-Brom-6-isopropyl-N-[(5-methyl-1,3,4-oxadiazol-2-yl-methyl)]-chloracetanilid
2,3-Dimethyl-N-[(5-methyl-1,3,4-oxadiazol-2-yl-methyl)]-chloracetanilid
2,5-Dimethyl-N-[(5-methyl-1,3,4-oxadiazol-2-yl-methyl)]-chloracetanilid
2,6-Diäthyl-N-[(5-äthyl-1,3,4-oxadiazol-2-yl-methyl)]-chloracetanilid
2-Äthyl-6-methyl-N-[(5-äthyl-1,3,4-oxadiazol-2-yl-methyl)]-chloracetanilid
2,6-Dimethyl-N-[(5-äthyl-1,3,4-oxadiazol-2-yl-methyl)]-chloracetanilid
2-tert.-Butyl-N-[(5-äthyl-1,3,4-oxadiazol-2-yl-methyl)]-chloracetanilid
2-Chlor-6-tert.-butyl-N-[(5-äthyl-1,3,4-oxadiazol-2-y-methyl)]-chloracetanilid
2,6-Diäthyl-N-[(5-isopropyl-1,3,4-oxadiazol-2-yl-methyl)]-chloracetanilid
2-Äthyl-6-methyl-N-[(5-isopropyl-1,3,4-oxadiazol-2-yl-methyl)]-chloracetanilid
2,6-Dimethyl-N-[(5-isopropyl-1,3,4-oxadiazol-2-yl-methyl)]-chloracetanilid
2-tert.-Butyl-N-[(5-isopropyl-1,3,4-oxadiazol-2-yl-methyl)]-chloracetanilid
2,6-Diäthyl-N-[(5-n-propyl-1,3,4-oxadiazol-2-yl-methyl)]-chloracetanilid
2-Äthyl-6-methyl-N-[(5-n-propyl-1,3,4-oxadiazol-2-yl-methyl)]-chloracetanilid
2,6-Dimethyl-N-[(5-n-propyl-1,3,4-oxadiazol-2-yl-methyl)]-chloracetanilid
2-tert.-Butyl-N-[(5-n-propyl-1,3,4-oxadiazol-2-yl-methyl)]-chloracetanilid
2,6-Diäthyl-N-[(5-phenyl-1,3,4-oxadiazol-2-yl-methyl)]-chloracetanilid
2,6-Diäthyl-N-[(5-phenyl-1,3,4-oxadiazol-2-yl-methyl)]-chloracetanilid
2-Äthyl-6-methyl-N-[(5-trichlormethyl-1,3,4-oxadiazol-2-yl-methyl)]-chloracetanilid
2,6-Dimethyl-N-[(5-trichlormethyl-1,3,4-oxadiazol-2-yl-methyl)]-chloracetanilid
2-tert.-Butyl-N-[(5-trichlormethyl-1,3,4-oxadiazol-2-yl-methyl)]-chloracetanilid
2-Chlor-6-tert.-butyl-N-[(5-trichlormethyl-1,3,4-oxadiazol-2-yl-methyl)]-chloracetanilid
2,6-Diäthyl-N-[(5-trifluormethyl-1,3,4-oxadiazol-2-yl-methyl)]-chloracetanilid
2-Äthyl-6-methyl-N-[(5-trifluormethyl-1,3,4-oxadiazol-2-yl-methyl)]-chloracetanilid
2,6-Dimethyl-N-[(5-trifluormethyl-1,3,4-oxadiazol-2-yl-methyl)]-chloracetanilid
2-tert.-Butyl-N-[(5-trifluormethyl-1,3,4-oxadiazol-2-yl-methyl)]-chloracetanilid
2-Chlor-6-tert.-butyl-N-[(5-trifluormethyl-1,3,4-oxadiazol-2-yl-methyl)]-chloracetanilid
2,6-Diäthyl-N-[(5-chlormethyl-1,3,4-oxadiazol-2-yl-methyl)]-chloracetanilid
2-Äthyl-6-methyl-N-[(5-chlormethyl-1,3,4-oxadiazol-2-yl-methyl)]-chloracetanilid
2,6-Dimethyl-N-[(5-chlormethyl-1,3,4-oxadiazol-2-yl-methyl)]-chloracetanilid
2,6-Diäthyl-N-[(5-chlor-1,3,4-oxadiazol-2-yl-methyl)]-chloracetanilid
2-Äthyl-6-methyl-N-[(5-chlor-1,3,4-oxadiazol-2-yl-methyl)]-chloracetanilid
2,6-Dimethyl-N-[(5-chlor-1,3,4-oxadiazol-2-yl-methyl)]-chloracetanilid
2,6-Diäthyl-N-[(5-brom-1,3,4-oxadiazol-2-yl-methyl)]-chloracetanilid
2-Äthyl-6-methyl-N-[(5-brom-1,3,4-oxadiazol-2-yl-methyl)]-chloracetanilid
2,6-Diäthyl-N-[(5-methoxy-1,3,4-oxadiazol-2-yl-methyl)]-chloracetanilid
2-Äthyl-6-methyl-N-[(5-methoxy-1,3,4-oxadiazol-2-yl-methyl)]-chloracetanilid
2,6-Dimethyl-N-[(5-methoxy-1,3,4-oxadiazol-2-yl-methyl)]-chloracetanilid
2-tert.-Butyl-N-[(5-methoxy-1,3,4-oxadiazol-2-yl-methyl)]-chloracetanilid
2,6-Diäthyl-N-[(5-methylthio-1,3,4-oxadiazol-2-yl-methyl)]-chloracetanilid
2-Äthyl-6-methyl-N-[(5-methylthio-1,3,4-oxadiazol-2-yl-methyl)]-chloracetanilid
2,6-Dimethyl-N-[(5-methylthio-1,3,4-oxadiazol-2-yl-methyl)]-chloracetanilid
2-tert.-Butyl-N-[(5-methylthio-1,3,4-oxadiazol-2-yl-methyl)]-chloracetanilid
2,6-Diäthyl-N-[(5-dimethylamino-1,3,4-oxadiazol-2-yl-methyl)]-chloracetanilid
2-Äthyl-6-methyl-N-[(5-dimethylamino-1,3,4-oxadiazol-2-yl-methyl)]-chloracetanilid
2,6-Diäthyl-N-[(5-benzyl-1,3,4-oxadiazol-2-yl-methyl)]-chloracetanilid
2-Äthyl-6-methyl-N-[(5-benzyl-1,3,4-oxadiazol-2-yl-methyl)]-chloracetanilid
2,6-Diäthyl-N-[(5-methyl-1,3,4-oxadiazol-2-yl-methyl)]-chloracetanilid
2-Äthyl-6-methyl-N-[(5-methyl-1,3,4-thiadiazol-2-yl-methyl)]-chloracetanilid
2,6-Dimethyl-N-[(5-methyl-1,3,4-thiadiazol-2-yl-methyl)]-chloracetanilid
2-tert.-Butyl-N-[(5-methyl-1,3,4-thiadiazol-2-yl-methyl)]-chloracetanilid
2-Chlor-6-tert.-butyl-N-[(5-methyl-1,3,4-thiadiazol-2-yl-methyl)]-chloracetanilid
2-Chlor-3-methyl-N-[(5-methyl-1,3,4-thiadiazol-2-yl-methyl)]-chloracetanilid
2-Chlor-6-methyl-N-[(5-methyl-1,3,4-thiadiazol-2-yl-methyl)]-chloracetanilid

2-Chlor-6-isopropyl-N-[(5-methyl-1,3,4-thiadiazol-2-yl-methyl)]-chloracetanilid
2-Isopropyl-N[(5-methyl-1,3,4-thiadiazol-2-yl-methyl)]-chloracetanilid
2-Äthyl-6-isopropyl-N-[(5-methyl-1,3,4-thiadiazol-2-yl-methyl)]-chloracetanilid
2-Brom-6-tert.-butyl-N-[(5-methyl-1,3,4-thiadiazol-2-yl-methyl)]-chloracetanilid
2-Brom-6-isopropyl-N-[(5-methyl-1,3,4-thiadiazol-2-yl-methyl)]-chloracetanilid
2,3-Dimethyl-N-[(5-methyl-1,3,4-thiadiazol-2-yl-methyl)]-chloracetanilid
2,5-Dimethyl-N-[(5-methyl-1,3,4-thiadiazol-2-yl-methyl)]-chloracetanilid
2,6-Diäthyl-N-[(5-äthyl-1,3,4-thiadiazol-2-yl-methyl)]-chloracetanilid
2-Äthyl-6-methyl-N-[(5-äthyl-1,3,4-thiadiazol-2-yl-methyl)]-chloracetanilid
2,6-Dimethyl-N-[(5-äthyl-1,3,4-thiadiazol-2-yl-methyl)]-chloracetanilid
2-tert.-Butyl-N-[(5-äthyl-1,3,4-thiadiazol-2-yl-methyl)]-chloracetanilid
2-Chlor-6-tert.-butyl-N-[(5-äthyl-1,3,4-thiadiazol-2-yl-methyl)]-chloracetanilid
2,6-Diäthyl-N-[(5-isopropyl-1,3,4-thiadiazol-2-yl-methyl)]-chloracetanilid
2-Äthyl-6-methyl-N-[(5-isopropyl-1,3,4-thiadiazol-2-yl-methyl)]-chloracetanilid
2,6-Dimethyl-N-[(5-isopropyl-1,3,4-thiadiazol-2-yl-methyl)]-chloracetanilid
2-tert.-Butyl-N-[(5-isopropyl-1,3,4-thiadiazol-2-yl-methyl)]-chloracetanilid
2,6-Diäthyl-N[(5-n-propyl-1,3,4-thiadiazol-2-yl-methyl)]-chloracetanilid
2-Äthyl-6-methyl-N-[(5-n-propyl-1,3,4-thiadiazol-2-yl-methyl)]-chloracetanilid
2,6-Dimethyl-N-[(5-n-propyl-1,3,4-thiadiazol-2-yl-methyl)]-chloracetanilid
2-tert.-Butyl-N-[(5-n-propyl-1,3,4-thiadiazol-2-yl-methyl)]-chloracetanilid
2,6-Diäthyl-N-[(5-phenyl-1,3,4-thiadiazol-2-yl-methyl)]-chloracetanilid
2,6-Diäthyl-N-[(5-phenyl-1,3,4-thiadiazol-2-yl-methyl)]-chloracetanilid
2-Äthyl-6-methyl-N-[(5-trichlormethyl-1,3,4-thiadiazol-2-yl-methyl)]-chloracetanilid
2,6-Dimethyl-N-[(5-trichlormethyl-1,3,4-thiadiazol-2-yl-methyl)]-chloracetanilid
2-tert.-Butyl-N-[(5-trichlormethyl-1,3,4-thiadiazol-2-yl-methyl)]-chloracetanilid
2-Chlor-6-tert.-butyl-N-[(5-trichlormethyl-1,3,4-thiadiazol-2-yl-methyl)]-chloracetanilid
2,6-Diäthyl-N-[(5-trifluormethyl-1,3,4-thiadiazol-2-yl-methyl)]-chloracetanilid
2-Äthyl-6-methyl-N-[(5-trifluormethyl-1,3,4-thiadiazol-2-yl-methyl)]-chloracetanilid
2,6-Dimethyl-N-[(5-trifluormethyl-1,3,4-thiadiazol-2-yl-methyl)]-chloracetanilid
2-tert.-Butyl-N-[(5-trifluormethyl-1,3,4-thiadiazol-2-yl-methyl)]-chloracetanilid
2-Chlor-6-tert.-butyl-N-[(5-trifluormethyl-1,3,4-thiadiazol-2-yl-methyl)]-chloracetanilid
2,6-Diäthyl-N-[(5-chlormethyl-1,3,4-thiadiazol-2-yl-methyl)]-chloracetanilid
2-Äthyl-6-methyl-N-[(5-chlormethyl-1,3,4-thiadiazol-2-yl-methyl)]-chloracetanilid
2,6-Dimethyl-N-[(5-chlormethyl-1,3,4-thiadiazol-2-yl-methyl)]-chloracetanilid
2,6-Diäthyl-N-[(5-chlor-1,3,4-thiadiazol-2-yl-methyl)]-chloracetanilid
2-Äthyl-6-methyl-N-[(5-chlor-1,3,4-thiadiazol-2-yl-methyl)]-chloracetanilid
2,6-Dimethyl-N-[(5-chlor-1,3,4-thiadiazol-2-yl-methyl)]-chloracetanilid
2,6-Diäthyl-N-[(5-brom-1,3,4-thiadiazol-2-yl-methyl)]-chloracetanilid
2-Äthyl-6-methyl-N-[(5-brom-1,3,4-thiadiazol-2-yl-methyl)]-chloracetanilid
2,6-Diäthyl-N-[(5-methoxy-1,3,4-thiadiazol-2-yl-methyl)]-chloracetanilid
2-Äthyl-6-methyl-N-[(5-methoxy-1,3,4-thiadiazol-2-yl-methyl)]-chloracetanilid
2,6-Dimethyl-N-[(5-methoxy-1,3,4-thiadiazol-2-yl-methyl)]-chloracetanilid
2-tert.-Butyl-N-[(5-methoxy-1,3,4-thiadiazol-2-yl-methyl)]-chloracetanilid
2,6-Diäthyl-N-[(5-methylthio-1,3,4-thiadiazol-2-yl-methyl)]-chloracetanilid
2-Äthyl-6-methyl-N-[(5-methylthio-1,3,4-thiadiazol-2-yl-methyl)]-chloracetanilid
2,6-Dimethyl-N-[(5-methylthio-1,3,4-thiadiazol-2-yl-methyl)]-chloracetanilid
2-tert.-Butyl-N-[(5-methylthio-1,3,4-thiadiazol-2-yl-methyl)]-chloracetanilid
2,6-Diäthyl-N-[(5-dimethylamino-1,3,4-thiadiazol-2-yl-methyl)]-chloracetanilid
2-Äthyl-6-methyl-N-[(5-dimethylamino-1,3,4-thiadiazol-2-yl-methyl)]-chloracetanilid
2,6-Diäthyl-N-[(5-benzyl-1,3,4-thiadiazol-2-yl-methyl)]-chloracetanilid
2-Äthyl-6-methyl-N-[(5-benzyl-1,3,4-thiadiazol-2-yl-methyl)]-chloracetanilid
2,6-Diäthyl-N-[(5-äthylthio-1,3,4-thiadiazol-2-yl-methyl)]-chloracetanilid
2-Äthyl-6-methyl-N-[(5-äthylthio-1,3,4-thiadiazol-2-yl-methyl)]-chloracetanilid
2,6-Dimethyl-N-[(5-äthylthio-1,3,4-thiadiazol-2-yl-methyl)]-chloracetanilid
2-tert.-Butyl-N-[(5-äthylthio-1,3,4-thiadiazol-2-yl-methyl)]-chloracetanilid
2,6-Diäthyl-N-[(5-allylthio-1,3,4-thiadiazol-2-yl-methyl)]-chloracetanilid
2-Äthyl-6-methyl-N-[(5-allylthio-1,3,4-thiadiazol-2-yl-methyl)]-chloracetanilid
2,6-Dimethyl-N-[(5-allylthio-1,3,4-thiadiazol-2-yl-methyl)]-chloracetanilid
2-tert.-Butyl-N-[(5-allylthio-1,3,4-thiadiazol-2-yl-methyl)]-chloracetanilid
2,6-Diäthyl-N-[(5-benzylthio-1,3,4-thiadiazol-2-yl-methyl)]-chloracetanilid
2-Äthyl-6-methyl-N-[(5-benzylthio-1,3,4-thiadiazol-2-yl-methyl)]-chloracetanilid
2,6-Dimethyl-N-[(5-benzylthio-1,3,4-thiadiazol-2-yl-methyl)]-chloracetanilid
2-tert.-Butyl-N-[(5-benzylthio-1,3,4-thiadiazol-2-yl-methyl)]-chloracetanilid
2,6-Diäthyl-N-[(5-isopropylthio-1,3,4-thiadiazol-2-yl-methyl)]-chloracetanilid
2-Äthyl-6-methyl-N-[(5-isopropylthio-1,3,4-thiadiazol-2-yl-methyl)]-chloracetanilid

2,6-Dimethyl-N-[(5-isopropylthio-1,3,4-thiadiazol-2-yl-methyl)]-chloracetanilid
2-tert.-Butyl-N-[(5-isopropylthio-1,3,4-thiadiazol-2-yl-methyl)]-chloracetanilid
2,6-Diäthyl-N-[(3,4-dimethyl-1,2,4-triazol-5-yl-methyl)]-chloracetanilid
2-Äthyl-6-methyl-N-[(3,4-dimethyl-1,2,4-triazol-5-yl-methyl)]-chloracetanilid
2,6-Dimethyl-N-[(3,4-dimethyl-1,2,4-triazol-5-yl-methyl)]-chloracetanilid
2-tert.-Butyl-N-[(3,4-dimethyl-1,2,4-triazol-5-yl-methyl)]-chloracetanilid
2,6-Diäthyl-N-[(3-äthyl-4-methyl-1,2,4-triazol-5-yl-methyl)]-chloracetanilid
2-Äthyl-6-methyl-N-[(3-äthyl-4-methyl-1,2,4-triazol-5-yl-methyl)]-chloracetanilid
2,6-Dimethyl-N-[(3-äthyl-4-methyl-1,2,4-triazol-5-yl-methyl)]-chloracetanilid
2-tert.-Butyl-N-[(3-äthyl-4-methyl-1,2,4-triazol-5-yl-methyl)]-chloracetanilid
2,6-Diäthyl-N-[(3-methyl-4-phenyl-1,2,4-triazol-5-yl-methyl)]-chloracetanilid
2-Äthyl-6-methyl-N-[(3-methyl-4-phenyl-1,2,4-triazol-5-yl-methyl)]-chloracetanilid
2,6-Dimethyl-N-[(3-methyl-4-phenyl-1,2,4-triazol-5-yl-methyl)]-chloracetanilid
2-tert.-Butyl-N-[(3-methyl-4-phenyl-1,2,4-triazol-5-yl-methyl)]-chloracetanilid
2,6-Diäthyl-N-[(3-methylmercapto-4-methyl-1,2,4-triazol-5-yl-methyl)]-chloracetanilid
2-Äthyl-6-methyl-N-[(3-methylmercapto-4-methyl-1,2,4-triazol-5-yl-methyl)]-chloracetanilid
2,6-Dimethyl-N-[(3-methylmercapto-4-methyl-1,2,4-triazol-5-yl-methyl)]-chloracetanilid
2-tert.-Butyl-N-[(3-methylmercapto-4-methyl-1,2,4-triazol-5-yl-methyl)]-chloracetanilid
2,6-Diäthyl-N-[(3-methoxy-4-methyl-1,2,4-triazol-5-yl-methyl)]-chloracetanilid
2-Äthyl-6-methyl-N-[(3-methoxy-4-methyl-1,2,4-triazol-5-yl-methyl)]-chloracetanilid
2,6-Dimethyl-N-[(3-methoxy-4-methyl-1,2,4-triazol-5-yl-methyl)]-chloracetanilid
2-tert.-Butyl-N-[(3-methoxy-4-methyl-1,2,4-triazol-5-yl-methyl)]-chloracetanilid
2,6-Diäthyl-N-[(3-methylmercapto-4-phenyl-1,2,4-triazol-5-yl-methyl)]-chloracetanilid
2-Äthyl-5-methyl-N-[(3-methylmercapto-4-phenyl-1,2,4-triazol-5-yl-methyl)]-chloracetanilid
2,6-Dimethyl-N-[(3-methylmercapto-4-phenyl-1,2,4-triazol-5-yl-methyl)]-chloracetanilid
2-tert.-Butyl-N-[(3-methylmercapto-4-phenyl-1,2,4-triazol-5-yl-methyl)]-chloracetanilid
2,6-Diäthyl-N-[(3-methoxy-4-phenyl-1,2,4-triazol-5-yl-methyl)]-chloracetanilid
2-Äthyl-6-methyl-N-[(3-methoxy-4-phenyl-1,2,4-triazol-5-yl-methyl)]-chloracetanilid
2,6-Dimethyl-N-[(3-methoxy-4-phenyl-1,2,4-triazol-5-yl-methyl)]-chloracetanilid
2-tert.-Butyl-N-[(3-methoxy-4-phenyl-1,2,3-triazol-5-yl-methyl)]-chloracetanilid
[sowie die entsprechenden Bromacetanilide]
2,6-Diäthyl-N-(1,3,4-oxadiazol-2-yl-methyl)-chloracetanilid
2-Äthyl-6-methyl-N-(1,3,4-oxadiazol-2-yl-methyl)-chloracetanilid
2,6-Dimethyl-N-(1,3,4-oxadiazol-2-yl-methyl)-chloracetanilid
2-tert.-Butyl-N-(1,3,4-oxadiazol-2-yl-methyl)-chloracetanilid
2,6-Diisopropyl-N-(1,3,4-oxadiazol-2-yl-methyl)-chloracetanilid
2,6-Diisopropyl-N-[(5-methyl-1,3,4-oxadiazol-2-yl-methyl)]-chloracetanilid
2-Chlor-6-tert.-butyl-N-(1,3,4-oxadiazol-2-yl-methyl)-chloracetanilid
2-Chlor-6-methyl-N-(1,3,4-oxadiazol-2-yl-methyl)-chloracetanilid
2,3-Dimethyl-N-(1,3,4-oxadiazol-2-yl-methyl)-chloracetanilid
2,5-Dimethyl-N-(1,3,4-oxadiazol-2-yl-methyl)-chloracetanilid
2,6-Diäthyl-N-(5-tert.-butyl-1,3,4-oxadiazol-2-yl-methyl)-chloracetanilid
2-Äthyl-6-methyl-N-(5-tert.-butyl-1,3,4-oxadiazol-2-yl-methyl)-chloracetanilid
2,6-Diisopropyl-N-(5-methyl-1,3,4-thiadiazol-2-yl-methyl)-chloracetanilid
2,6-Diäthyl-N-(5-methyl-1,3,4-thiadiazol-2-yl-methyl)-chloracetanilid
2,6-Diäthyl-N-(1,3,4-thiadiazol-2-yl-methyl)-chloracetanilid
2-Äthyl-6-methyl-N-(1,3,4-thiadiazol-2-yl-methyl)-chloracetanilid
2,6-Dimethyl-N-(1,3,4-thiadiazol-2-yl-methyl)-chloracetanilid
2-tert.-Butyl-N-(1,3,4-thiadiazol-2-yl-methyl)-chloracetanilid
2,6-Diisopropyl-N-(1,3,4-thiadiazol-2-yl-methyl)-chloracetanilid
2-Chlor-6-methyl-N-(1,3,4-thiadiazol-2-yl-methyl)-chloracetanilid
2-Chlor-6-tert.-butyl-N-(1,3,4-thiadiazol-2-yl-methyl)-chloracetanilid
2,3-Dimethyl-N-(1,3,4-thiadiazol-2-yl-methyl)-chloracetanilid
2,5-Dimethyl-N-(1,3,4-thiadiazol-2-yl-methyl)-chloracetanilid
2,6-Diäthyl-N-(4-methyl-1,2,4-triazol-5-yl-methyl)-chloracetanilid
2,6-Dimethyl-N-(4-methyl-1,2,4-triazol-5-yl-methyl)-chloracetanilid
2-Äthyl-6-methyl-N-(4-methyl-1,2,4-triazol-5-yl-methyl)-chloracetanilid
2-tert.-Butyl-N-(4-methyl-1,2,4-triazol-5-yl-methyl)-chloracetanilid
2,6-Diisopropyl-N-(4-methyl-1,2,4-triazol-5-yl-methyl)-chloracetanilid
sowie die entsprechenden Bromacetanilide.

Verwendet man 2,6-Diäthyl-N-(3-methylthio-4-methyl-1,2,4-triazol-5-yl-methyl)-anilin und Chloracetylchlord als Ausgangsstoffe, so kann der Reaktionsablauf durch das folgende Formelschema wiedergegeben werden:

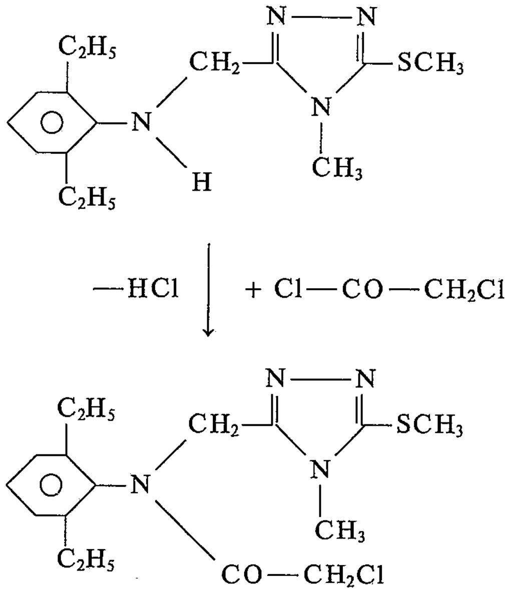

Die bei der Durchführung des erfindungsgemäßen Verfahrens als Ausgangsstoffe benötigten N-Azolylalkylaniline sind durch die Formel (II) allgemein definiert. In dieser Formel stehen A, R, $R^1$, X, Y und n vorzugsweise für diejenigen Reste, die bereits im Zusammenhang mit der Beschreibung der erfindungsgemäßen Stoffe der Formel (I) vorzugsweise für diese Reste genannt wurden. Die Reste $R^2$ und $R^3$ stehen ebenfalls vorzugsweise für diejenigen Reste, die bereits im Zusammenhang mit der Beschreibung der erfindungsgemäßen Stoffe der Formel (I) vorzugsweise für die Reste $R^2$ und $R^3$ genannt wurden.

Als Beispiele für die N-Azolylalkylaniline der Formel (II) seien genannt:

2,6-Diäthyl-N-(5-methyl-1,3,4-oxadiazol-2-yl-methyl)-anilin
2-Äthyl-6-methyl-N-(5-methyl-1,3,4-oxadiazol-2-yl-methyl)-anilin
2,6-Dimethyl-N-(5-methyl-1,3,4-oxadiazol-2-yl-methyl)-anilin
2-tert.-Butyl-N-(5-methyl-1,3,4-oxadiazol-2-yl-methyl)-anilin
2-Chlor-6-tert.-butyl-N-(5-methyl-1,3,4-oxadiazol-2-yl-methyl)-anilin
2-Chlor-3-methyl-N-(5-methyl-1,3,4-oxadiazol-2-yl-methyl)-anilin
2-Chlor-5-methyl-N-(5-methyl-1,3,4-oxadiazol-2-yl-methyl)-anilin
2-Chlor-6-isopropyl-N-(5-methyl-1,3,4-oxadiazol-2-yl-methyl)-anilin
2-Isopropyl-N-(5-methyl-1,3,4-oxadiazol-2-yl-methyl)-anilin
2-Äthyl-6-isopropyl-N-(5-methyl-1,3,4-oxadiazol-2-yl-methyl)-anilin
2-Brom-6-tert.-butyl-N-(5-methyl-1,3,4-oxadiazol-2-yl-methyl)-anilin
2-Brom-6-isopropyl-N-(5-methyl-1,3,4-oxadiazol-2-yl-methyl)-anilin
2,3-Dimethyl-N-(5-methyl-1,3,4-oxadiazol-2-yl-methyl)-anilin
2,5-Dimethyl-N-(5-methyl-1,3,4-oxadiazol-2-yl-methyl)-anilin
2,6-Diäthyl-N-(5-äthyl-1,3,4-oxadiazol-2-yl-methyl)-anilin
2-Äthyl-6-methyl-N-(5-äthyl-1,3,4-oxadiazol-2-yl-methyl)-anilin
2,6-Dimethyl-N-(5-äthyl-1,3,4-oxadiazol-2-yl-methyl)-anilin
2-tert.-Butyl-N-(5-äthyl-1,3,4-oxadiazol-2-yl-methyl)-anilin
2-Chlor-6-tert.-butyl-N-(5-äthyl-1,3,4-oxadiazol-2-yl-methyl)-anilin
2,6-Diäthyl-N-(5-isopropyl-1,3,4-oxadiazol-2-yl-methyl)-anilin
2-Äthyl-6-methyl-N-(5-isopropyl-1,3,4-oxadiazol-2-yl-methyl)-anilin
2,6-Dimethyl-N-(5-isopropyl-1,3,4-oxadiazol-2-yl-methyl)-anilin
2-tert.-Butyl-N-(5-isopropyl-1,3,4-oxadiazol-2-yl-methyl)-anilin
2,6-Diäthyl-N-(5-n-propyl-1,3,4-oxadiazol-2-yl-methyl)-anilin
2-Äthyl-6-methyl-N-(5-n-propyl-1,3,4-oxadiazol-2-yl-methyl)-anilin
2,6-Dimethyl-N-(5-n-propyl-1,3,4-oxadiazol-2-yl-methyl)-anilin
2-tert.-Butyl-N-(5-n-propyl-1,3,4-oxadiazol-2-yl-methyl)-anilin
2,6-Diäthyl-N-(5-phenyl-1,3,4-oxadiazol-2-yl-methyl)-anilin
2,6-Diäthyl-N-(5-phenyl-1,3,4-oxadiazol-2-yl-methyl)-anilin
2-Äthyl-6-methyl-N-(5-trichlormethyl-1,3,4-oxadiazol-2-yl-methyl)-anilin
2,6-Dimethyl-N-(5-trichlormethyl-1,3,4-oxadiazol-2-yl-methyl)-anilin
2-tert.-Butyl-N-(5-trichlormethyl-1,3,4-oxadiazol-2-yl-methyl)-anilin
2-Chlor-6-tert.-butyl-N-(5-trichlormethyl-1,3,4-oxadiazol-2-yl-methyl)-anilin
2,6-Diäthyl-N-(5-trifluormethyl-1,3,4-oxadiazol-2-yl-methyl)-anilin

2-Äthyl-6-methyl-N-(5-trifluormethyl-1,3,4-oxadiazol-2-yl-methyl)-anilin
2,6-Dimethyl-N-(5-trifluormethyl-1,3,4-oxadiazol-2-yl-methyl)-anilin
2-tert.-Butyl-N-(5-trifluormethyl-1,3,4-oxadiazol-2-yl-methyl)-anilin
2-Chlor-6-tert.-butyl-N-(5-trifluormethyl-1,3,4-oxadiazol-2-yl-methyl)-anilin
2,6-Diäthyl-N-(5-chlormethyl-1,3,4-oxadiazol-2-yl-methyl)-anilin
2-Äthyl-6-methyl-N-(5-chlormethyl-1,3,4-oxadiazol-2-yl-methyl)-anilin
2,6-Dimethyl-N-(5-chlormethyl-1,3,4-oxadiazol-2-yl-methyl)-anilin
2,6-Diäthyl-N-(5-chlor-1,3,4-oxadiazol-2-yl-methyl)-anilin
2-Äthyl-6-methyl-N-(5-chlor-1,3,4-oxadiazol-2-yl-methyl)-anilin
2,6-Dimethyl-N-(5-chlor-1,3,4-oxadiazol-2-yl-methyl)-anilin
2,6-Diäthyl-N-(5-brom-1,3,4-oxadiazol-2-yl-methyl)-anilin
2-Äthyl-6-methyl-N-(5-brom-1,3,4-oxadiazol-2-yl-methyl)-anilin
2,6-Diäthyl-N-(5-methoxy-1,3,4-oxadiazol-2-yl-methyl)-anilin
2-Äthyl-6-methyl-N-(5-methoxy-1,3,4-oxadiazol-2-yl-methyl)-anilin
2,6-Dimethyl-N-(5-methoxy-1,3,4-oxadiazol-2-yl-methyl)-anilin
2-tert.-Butyl-N-(5-methoxy-1,3,4-oxadiazol-2-yl-methyl)-anilin
2,6-Diäthyl-N-(5-methylthio-1,3,4-oxodiazol-2-yl-methyl)-anilin
2-Äthyl-6-methyl-N-(5-methylthio-1,3,4-oxadiazol-2-yl-methyl)-anilin
2,6-Dimethyl-N-(5-methylthio-1,3,4-oxadiazol-2-yl-methyl)-anilin
2-tert.-Butyl-N-(5-methylthio-1,3,4-oxadiazol-2-yl-methyl)-anilin
2,6-Diäthyl-N-(5-dimethylamino-1,3,4-oxodiazol-2-yl-methyl)-anilin
2-Äthyl-6-methyl-N-(5-dimethylamino-1,3,4-oxadiazol-2-yl-methyl)-anilin
2,6-Diäthyl-N-(5-benzyl-1,3,4-oxadiazol-2-yl-methyl)-anilin
2-Äthyl-6-methyl-N-(5-benzyl-1,3,4-oxadiazol-2-yl-methyl)-anilin
2,6-Diäthyl-N-(5-äthylthio-1,3,4-thiadiazol-2-yl-methyl)-anilin
2-Äthyl-6-methyl-N-(5-äthylthio-1,3,4-thiadiazol-2-yl-methyl)-anilin
2,6-Dimethyl-N-(5-äthylthio-1,3,4-thiadiazol-2-yl-methyl)-anilin
2-tert.-Butyl-N-(5-äthylthio-1,3,4-thiadiazol-2-yl-methyl)-anilin
2-Chlor-6-tert.-butyl-N-(5-methyl-1,3,4-thiadiazol-2-yl-methyl)-anilin
2-Chlor-3-methyl-N-(5-methyl-1,3,4-thiadiazol-2-yl-methyl)-anilin
2-Chlor-5-methyl-N-(5-methyl-1,3,4-thiadiazol-2-yl-methyl)-anilin
2-Chlor-6-isopropyl-N-(5-methyl-1,3,3-thiadiazol-2-yl-methyl)-anilin
2-Isopropyl-N-(5-methyl-1,3,4-thiadiazol-2-yl-methyl)-anilin
2-Äthyl-6-isopropyl-N-(5-methyl-1,3,4-thiadiazol-2-yl-methyl)-anilin
2-Brom-6-tert.-butyl-N-(5-methyl-1,3,4-thiadiazol-2-yl-methyl)-anilin
2-Brom-6-isopropyl-N-(5-methyl-1,3,4-thiadiazol-2-yl-methyl)-anilin
2,3-Dimethyl-N-(5-methyl-1,3,4-thiadiazol-2-yl-methyl)-anilin
2,5-Dimethyl-N-(5-methyl-1,3,4-thiadiazol-2-yl-methyl)-anilin
2,6-Diäthyl-N-(5-äthyl-1,3,4-thiadiazol-2-yl-methyl)-anilin
2-Äthyl-6-methyl-N-(5-äthyl-1,3,4-thiadiazol-2-yl-methyl)-anilin
2,6-Dimethyl-N-(5-äthyl-1,3,4-thiadiazol-2-yl-methyl)-anilin
2-tert.-Butyl-N-(5-äthyl-1,3,4-thiadiazol-2-yl-methyl)-anilin
2-Chlor-6-tert.-butyl-N-(5-äthyl-1,3,4-thiadiazol-2-yl-methyl)-anilin
2,6-Diäthyl-N-(5-isopropyl-1,3,4-thiadiazol-2-yl-methyl)-anilin
2-Äthyl-6-methyl-N-(5-isopropyl-1,3,4-thiadiazol-2-yl-methyl)-anilin
2,6-Dimethyl-N-(5-isopropyl-1,3,4-thiadiazol-2-yl-methyl)-anilin
2-tert.-Butyl-N-(5-isopropyl-1,3,4-thiadiazol-2-yl-methyl)-anilin
2,6-Diäthyl-N-(5-n-propyl-1,3,4-thiadiazol-2-yl-methyl)-anilin
2-Äthyl-6-methyl-N-(5-n-propyl-1,3,4-thiadiazol-2-yl-methyl)-anilin
2,6-Dimethyl-N-(5-n-propyl-1,3,4-thiadiazol-2-yl-methyl)-anilin
2-tert.-Butyl-N-(5-n-propyl-1,3,4-thiadiazol-2-yl-methyl)-anilin
2,6-Diäthyl-N-(5-phenyl-1,3,4-thiadiazol-2-yl-methyl)-anilin
2,6-Diäthyl-N-(5-phenyl-1,3,4-thiadiazol-2-yl-methyl)-anilin
2-Äthyl-6-methyl-N-(5-trichlormethyl-1,3,4-thiadiazol-2-yl-methyl)-anilin
2,6-Dimethyl-N-(5-trichlormethyl-1,3,4-thiadiazol-2-yl-methyl)-anilin
2-tert.-Butyl-N-(5-trichlormethyl-1,3,4-thiadiazol-2-yl-methyl)-anilin
2-Chlor-6-tert.-butyl-N-(5-trichlormethyl-1,3,4-thiadiazol-2-yl-methyl)-anilin
2,6-Diäthyl-N-(5-trifluormethyl-1,3,4-thiadiazol-2-yl-methyl)-anilin
2-Äthyl-6-methyl-N-(5-trifluormethyl-1,3,4-thiadiazol-2-yl-methyl)-anilin
2,6-Dimethyl-N-(5-trifluormethyl-1,3,4-thiadiazol-2-yl-methyl)-anilin
2-tert.-Butyl-N-(5-trifluormethyl-1,3,4-thiadiazol-2-yl-methyl)-anilin
2-Chlor-6-tert.-butyl-N-(5-trifluormethyl-1,3,4-thiadiazol-2-yl-methyl)-anilin
2,6-Diäthyl-N-(5-chlormethyl-1,3,4-thiadiazol-2-yl-methyl)-anilin
2-Äthyl-6-methyl-N-(5-chlormethyl-1,3,4-thiadiazol-2-yl-methyl)-anilin
2,6-Dimethyl-N-(5-chlormethyl-1,3,4-thiadiazol-2-yl-methyl)-anilin

2,6-Diäthyl-N-(5-chlor-1,3,4-thiadiazol-2-yl-methyl)-anilin
2-Äthyl-6-methyl-N-(5-chlor-1,3,4-thiadiazol-2-yl-methyl)-anilin
2,6-Dimethyl-N-(5-chlor-1,3,4-thiadiazol-2-yl-methyl)-anilin
2,6-Diäthyl-N-(5-brom-1,3,4-thiadiazol-2-yl-methyl)-anilin
2-Äthyl-6-methyl-N-(5-brom-1,3,4-thiadiazol-2-yl-methyl)-anilin
2,6-Diäthyl-N-(5-methoxy-1,3,4-thiadiazol-2-yl-methyl)-anilin
2-Äthyl-6-methyl-N-(5-methoxy-1,3,4-thiadiazol-2-yl-methyl)-anilin
2,6-Dimethyl-N-(5-methoxy-1,3,4-thiadiazol-2-yl-methyl)-anilin
2-tert.-Butyl-N-(5-methoxy-1,3,4-thiadiazol-2-yl-methyl)-anilin
2,6-Diäthyl-N-(5-methylthio-1,3,4-thiadiazol-2-yl-methyl)-anilin
2-Äthyl-6-methyl-N-(5-methylthio-1,3,4-thiadiazol-2-yl-methyl)-anilin
2,6-Dimethyl-N-(5-methylthio-1,3,4-thiadiazol-2-yl-methyl)-anilin
2-tert.-Butyl-N-(5-methylthio-1,3,4-thiadiazol-2-yl-methyl)-anilin
2,6-Diäthyl-N-(5-dimethylamino-1,3,4-thiadiazol-2-yl-methyl)-anilin
2-Äthyl-6-methyl-N-(5-dimethylamino-1,3,4-thiadiazol-2-yl-methyl)-anilin
2,6-Diäthyl-N-(5-benzyl-1,3,4-thiadiazol-2-yl-methyl)-anilin
2-Äthyl-6-methyl-N-(5-benzyl-1,3,4-thiadiazol-2-yl-methyl)-anilin
2,6-Diäthyl-N-(5-äthylthio-1,3,4-thiadiazol-2-yl-methyl)-anilin
2-Äthyl-6-methyl-N-(5-äthylthio-1,3,4-thiadiazol-2-yl-methyl)-anilin
2,6-Dimethyl-N-(5-äthylthio-1,3,4-thiadiazol-2-yl-methyl)-anilin
2-tert.-Butyl-N-(5-äthylthio-1,3,4-thiadiazol-2-yl-methyl)-anilin
2,6-Diäthyl-N-(5-allylthio-1,3,4-thiadiazol-2-yl-methyl)-anilin
2-Äthyl-6-methyl-N-(5-allylthio-1,3,4-thiadiazol-2-yl-methyl)-anilin
2,6-Dimethyl-N-(5-allylthio-1,3,4-thiadiazol-2-yl-methyl)-anilin
2-tert.-Butyl-N-(5-allylthio-1,3,4-thiadiazol-2-yl-methyl)-anilin
2,6-Diäthyl-N-(5-benzylthio-1,3,4-thiadiazol-2-yl-methyl)-anilin
2-Äthyl-6-methyl-N-(5-benzylthio-1,3,4-thiadiazol-2-yl-methyl)-anilin
2,6-Dimethyl-N-(5-benzylthio-1,3,4-thiadiazol-2-yl-methyl)-anilin
2-tert.-Butyl-N-(5-benzylthio-1,3,4-thiadiazol-2-yl-methyl)-anilin
2,6-Diäthyl-N-(5-isopropylthio-1,3,4-thiadiazol-2-yl-methyl)-anilin
2-Äthyl-6-methyl-N-(5-isopropylthio-1,3,4-thiadiazol-2-yl-methyl)-anilin
2,6-Dimethyl-N-(5-isopropylthio-1,3,4-thiadiazol-2-yl-methyl)-anilin
2-tert.-Butyl-N-(5-isopropylthio-1,3,4-thiadiazol-2-yl-methyl)-anilin
2,6-Diäthyl-N-(3,4-dimethyl-1,2,4-triazol-5-yl-methyl)-anilin
2-Äthyl-6-methyl-N-(3,4-dimethyl-1,2,4-triazol-5-yl-methyl)-anilin
2,6-Dimethyl-N-(3,4-dimethyl-1,2,4-triazol-5-yl-methyl)-anilin
2-tert.-Butyl-N-(3,4-dimethyl-1,2,4-triazol-5-yl-methyl)-anilin
2,6-Diäthyl-N-(3-äthyl-4-methyl-1,2,4-triazol-5-yl-methyl)-anilin
2-Äthyl-6-methyl-N-(3-äthyl-4-methyl-1,2,4-triazol-5-yl-methyl)-anilin
2,6-Dimethyl-N-(3-äthyl-4-methyl-1,2,4-triazol-5-yl-methyl)-anilin
2-tert.-Butyl-N-(3-äthyl-4-methyl-1,2,4-triazol-5-yl-methyl)-anilin
2,6-Diäthyl-N-(3-methyl-4-phenyl-1,2,4-triazol-5-yl-methyl)-anilin
2-Äthyl-6-methyl-N-(3-methyl-4-phenyl-1,2,4-triazol-5-yl-methyl)-anilin
2,6-Dimethyl-N-(3-methyl-4-phenyl-1,2,4-triazol-5-yl-methyl)-anilin
2-tert.-Butyl-N-(3-methyl-4-phenyl-1,2,4-triazol-5-yl-methyl)-anilin
2,6-Diäthyl-N-(3-methylmercapto-4-methyl-1,2,4-triazol-5-yl-methyl)-anilin
2-Äthyl-6-methyl-N-(3-methylmercapto-4-methyl-1,2,4-triazol-5-yl-methyl)-anilin
2,6-Dimethyl-N-(3-methylmercapto-4-methyl-1,2,4-triazol-5-yl-methyl)-anilin
2-tert.-Butyl-N-(3-methylmercapto-4-methyl-1,2,4-triazol-5-yl-methyl)-anilin
2,6-Diäthyl-N-(3-methoxy-4-methyl-1,2,4-triazol-5-yl-methyl)-anilin
2-Äthyl-6-methyl-N-(3-methoxy-4-methyl-1,2,4-triazol-5-yl-methyl)-anilin
2,6-Dimethyl-N-(3-methoxy-4-methyl-1,2,4-triazol-5-yl-methyl)-anilin
2-tert.-Butyl-N-(3-methoxy-4-methyl-1,2,4-triazol-5-yl-methyl)-anilin
2,6-Diäthyl-N-(3-methylmercapto-4-phenyl-1,2,4-triazol-5-yl-methyl)-anilin
2-Äthyl-5-methyl-N-(3-methylmercapto-4-phenyl-1,2,4-triazol-5-yl-methyl)-anilin
2,6-Dimethyl-N-(3-methylmercapto-4-phenyl-1,2,4-triazol-5-yl-methyl)-anilin
2-tert.-Butyl-N-(3-methylmercapto-4-phenyl-1,2,4-triazol-5-methyl)-anilin
2,6-Diäthyl-N-(3-methoxy-4-phenyl-1,2,4-triazol-5-yl-methyl)-anilin
2-Äthyl-6-methyl-N-(3-methoxy-4-phenyl-1,2,4-triazol-5-yl-methyl)-anilin
2,6-Dimethyl-N-(3-methoxy-4-phenyl-1,2,4-triazol-5-yl-methyl)-anilin
2-tert.-Butyl-N-(3-methoxy-4-phenyl-1,2,4-triazol-5-yl-methyl)-anilin
2,6-Diisopropyl-N-(5-methyl-1,3,4-oxadiazol-2-yl-methyl)-anilin
2-Chlor-6-methyl-N-(5-methyl-1,3,4-oxadiazol-2-yl-methyl)-anilin
2,6-Diäthyl-N-(1,3,4-oxadiazol-2-yl-methyl)-anilin
2,6-Dimethyl-N-(1,3,4-oxadiazol-2-yl-methyl)-anilin

2-Äthyl-6-methyl-N-(1,3,4-oxadiazol-2-yl-methyl)-anilin
2-tert.-Butyl-N-(1,3,4-oxadiazol-2-yl-methyl)-anilin
2,6-Diisopropyl-N-(1,3,4-oxadiazol-2-yl-methyl)-anilin
2-Chlor-6-tert.-butyl-N-(1,3,4-oxadiazol-2-yl-methyl)-anilin
2,6-Diäthyl-N-(5-methyl-1,3,4-thiadiazol-2-yl-methyl)-anilin
2-Äthyl-6-methyl-N-(5-methyl-1,3,4-thiadiazol-2-yl-methyl)-anilin
2,6-Dimethyl-N-(5-methyl-1,3,4-thiadiazol-2-yl-methyl)-anilin
2-tert.-Butyl-N-(5-methyl-1,3,4-thiadiazol-2-yl-methyl)-anilin
2,6-Diäthyl-N-(1,3,4-thiadiazol-2-yl-methyl)-anilin
2,6-Diisopropyl-N-(1,3,4-thiadiazol-2-yl-methyl)-anilin
2-Äthyl-6-methyl-N-(1,3,4-thiadiazol-2-yl-methyl)-anilin
2,6-Dimethyl-N-(1,3,4-thiadiazol-2-yl-methyl)-anilin
2-Chlor-6-tert.-butyl-N-(1,3,4-thiadiazol-2-yl-methyl)-anilin
2-tert.-Butyl-N-(1,3,4-thiadiazol-2-yl-methyl)-anilin

Die N-Azolylalkylaniline der Formel (II) sind noch nicht bekannt. Man erhält sie, wenn man

a) Aniline der Formel

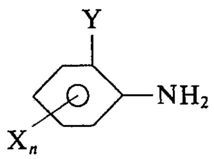

(IV)

in welcher
X, Y und n die oben angegebene Bedeutung haben,

mit Azol-Derivaten der Formel

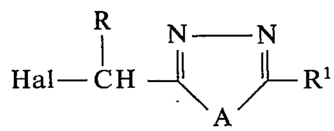

(V)

in welcher
A, R und $R^1$ die oben angegebene Bedeutung haben und
Hal für Chlor oder Brom steht,

in Gegenwart eines Säurebinders, wie beispielsweise Kalium- oder Natriumcarbonat, und in Gegenwart eines inerten organischen Lösungsmittels, wie beispielsweise Dimethylformamid oder Toluol, bei Temperaturen zwischen 20 und 160°C umsetzt, wobei vorzugsweise ein Überschuß an Anilin der Formel (IV) eingesetzt wird (vergleiche auch Herstellungsbeispiele), oder

b Hydrazin-Derivate der Formel

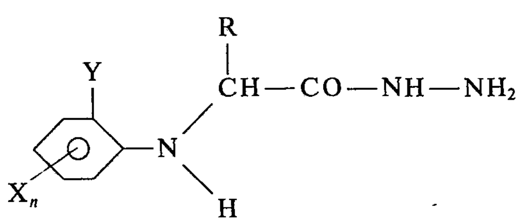

(VI)

in welcher
R, X, Y und n die oben angegebene Bedeutung haben,

mit Isocyanaten bzw. Senfölen der Formel

$$R^2—N=C=B \quad (VII)$$

in welcher
B für Sauerstoff oder Schwefel steht und
$R^2$ die oben angegebene Bedeutung hat,

in Gegenwart eines organischen Lösungsmittels, wie beispielsweise eines Alkohols, Äthers oder Kohlenwasserstoffs, bei Temperaturen zwischen 0 und 80°C umsetzt, die entstehenden Verbindungen der Formel

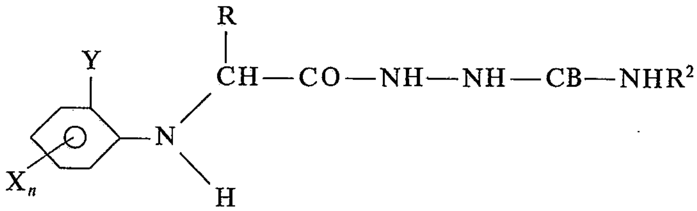

(VIII)

in welcher
B, R, $R^2$, X, Y und n die oben angegebene Bedeutung haben,

in Gegenwart einer starken Base, wie beispielsweise Natron-oder Kalilauge, und in Gegenwart eines Lösungsmittels, wie beispielsweise Äthanol oder Wasser bei Temperaturen zwischen 20 und 100°C cyclisiert und die entstehenden Triazolone bzw. Triazolthione der Formel

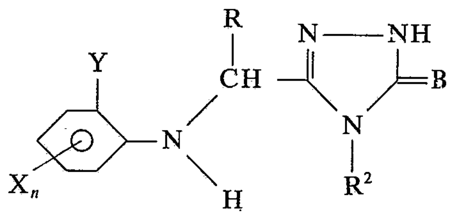

(IX)

in welcher
B, R, $R^2$, X, Y und n die oben angegebene Bedeutung haben,

mit Halogeniden der Formel

$$Hal—R^4 \qquad (X)$$

in welcher
Hal für Chlor oder Brom steht und
$R^4$ für die Reste des Substituenten $R^3$ steht, wobei Wasserstoff ausgenommen ist,

in Gegenwart einer starken Base, wie beispielsweise Natronlauge, und in Gegenwart eines inerten organischen Lösungsmittels, wie beispielsweise Toluol oder Methylenchlorid, bei Temperaturen zwischen 20 und 80°C umsetzt, wobei auch Phasentransferkatalysiert und mit anderen Alkylierungsreagenzien, wie beispielsweise Dimethylsulfat, gearbeitet werden kann (vergleiche auch Herstellungsbeispiele), oder

c) Hydrazin-Derivate der Formel (VI) mit Ameisensäure oder Säurechloriden bzw. Säureanhydriden der Formeln

$$R^5—CO—Cl \qquad (XI\,a)$$

bzw.

$$(R^5—CO—)_2O \qquad (XI\,b)$$

in welchen
$R^5$ für Alkyl mit 1 bis 4 Kohlenstoffatomen, Halogenalkyl mit bis zu 5 gleichen oder verschiedenen Halogenatomen, Alkenyl mit 2 bis 4 Kohlenstoffatomen, Alkinyl mit 2 bis 4 Kohlenstoffatomen, Cycloalkyl mit 5 bis 7 Kohlenstoffatomen, für gegebenenfalls durch Halogen, Alkyl mit 1 bis 4 Kohlenstoffatomen, Alkoxy mit 1 oder 2 Kohlenstoffatomen, Alkylthio mit 1 bis 2 Kohlenstoffatomen, Cyano, Nitro und/oder Halogenalkyl mit bis zu 2 Kohlenstoffatomen und bis zu 5 gleichen oder verschiedenen Halogenatomen substituiertes Aryl mit 6 bis 10 Kohlenstoffatomen sowie für Aralkyl mit 6 bis 10 Kohlenstoffatomen im Arylteil und 1 bis 4 Kohlenstoffatomen im Alkylteil steht, wobei der Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen,

in Gegenwart eines inerten organischen Lösungsmittels, wie eines Äthers, Kohlenwasserstoffes oder Halogenkohlenwasserstoffes, bei Temperaturen zwischen 0°C und 50°C umsetzt und die entstehenden Verbindungen der Formel

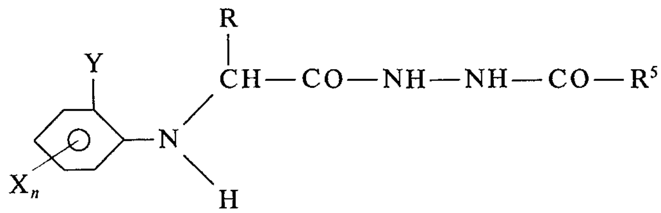

(XII)

in welcher
R, $R^5$, X, Y und n die oben angegebene Bedeutung haben,

entweder mit Diphosphorpentasulfid in an sich bekannter Weise (vgl. Chem. Ber. 32. 797 [1899] und J. prakt. Chemie 69, 145 [1904] zu Thiadiazol-Derivaten cyclisiert, oder ebenfalls in bekannter Weise mit üblichen wasserabspaltenden Reagenzien zu Oxadiazol-Derivaten umsetzt (vgl. hierzu Elderfield, Heterocyclic Compounds, Vol. 7[1961]) oder

d) Hydrazin-Derivate der Formel (VI) mit Nitrilen der Formel

$$R^8—C \equiv N \qquad (XIII)$$

in welcher
$R^6$ für Alkyl mit 1 bis 4 Kohlenstoffatomen, Halogenalkyl mit 1 bis 4 Kohlenstoffatomen und 1 bis 5 Halogenatomen oder für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Nitro substituiertes Aryl mit 6 bis 10 Kohlenstoffatomen steht,

in an sich bekannter Weise zu Triazol-Derivaten umsetzt (vergleiche Chem. Ber. 96. 1064 [1963]), oder

e) Hydrazin-Derivate der Formel (VI) mit Iminoäthern der Formel

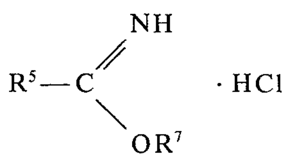

(XIV)

in welcher
$R^5$ die oben angegebene Bedeutung hat und
$R^7$ für Methyl oder Äthyl steht,

in an sich bekannter Weise unter Rückfluß und in Gegenwart eines inerten organischen Lösungsmittels, wie beispielsweise Äthanol, zu Oxadiazol-Derivaten umsetzt, oder

f) die Aniline der Formel (IV) mit Azol-aldehyden der Formel

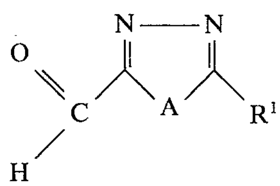

(XV)

in welcher
$R^1$ die oben angegebene Bedeutung hat,

in Gegenwart eines inerten organischen Lösungsmittels, wie beispielsweise Toluol, bei Temperaturen zwischen 80 und 120°C umsetzt und die entstehenden Verbindungen der Formel

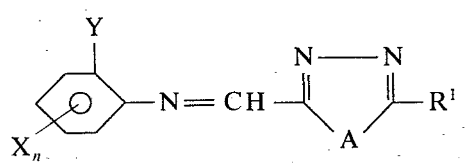

in welcher A, $R^1$, X, Y und n die oben angegebene Bedeutung haben,

in allgemein bekannter Weise reduziert; z. B. durch Umsetzung mit komplexen Hydriden, wie Natriumborhydrid, gegebenenfalls in Gegenwart eines polaren organischen Lösungsmittels, wie Methanol, bei Temperaturen zwischen 0 und 80° C.

Die Ausgangsstoffe der Formel (IV) sind allgemein bekannte Verbindungen der organischen Chemie. Als Beispiele seien genannt:

2-Methylanilin, 2-Äthylanilin, 2-Propylanilin, 2-Isopropylanilin, 2-Butylanilin, 2-Isobutylanilin, 2-sec.-Butylanilin, 2-tert.-Butylanilin, 2,6-Dimethylanilin, 2,6-Diäthylanilin, 2-Äthyl-6-methylanilin, 2,6-Diisopropylanilin, 2,3-Dimethylanilin, 2,4-Dimethylanilin, 2,5-Dimethylanilin, 2-Äthyl-3-methylanilin, 2-Äthyl-4-methylanilin, 2-Äthyl-5-methylanilin, 2,4,6-Trimethylanilin, 2,4,5-Trimethylanilin, 2-Äthyl-4,6-dimethylanilin, 2,6-Diäthyl-4-methylanilin, 2,6-Diisopropyl-4-methylanilin, 2-Chlor-6-methylanilin, 5-Chlor-2-methylanilin, 3-Chlor-2-methylanilin, 4-Chlor-2-methylanilin, 2-Chlor-6-tert.-butylanilin.

Die Ausgangsstoffe der Formel (V) sind bekannt oder lassen sich nach allgemein üblichen und bekannten Heterocyclensynthesen herstellen (vergleiche hierzu u. a. Helv. Chim. Acta 55, 1979ff. [1972]; Chem. Ber. 32, 797ff. [1899] sowie 96, 1049ff. [1963]).
Als Beispiele seien genannt:

2-Chlormethyl-5-methyl-1,3,4-oxadiazol
2-Chlormethyl-5-äthyl-1,3,4-oxadiazol
2-Chlormethyl-5-isopropyl-1,3,4-oxadiazol
2-Chlormethyl-5-trifluormethyl-1,3,4-oxadiazol
2-Chlormethyl-5-trichlormethyl-1,3,4-oxadiazol
2-Chlormethyl-5-allyl-1,3,4-oxadiazol
2-Chlormethyl-5-cyclohexyl-1,3,4-oxadiazol
2-Chlormethyl-5-benzyl-1,3,4-oxadiazol
2-Chlormethyl-5-methoxy-1,3,4-oxadiazol
2-Chlormethyl-5-allyloxy-1,3,4-oxadiazol
2-Chlormethyl-5-(2-fluorbenzyloxy)-1,3,4-oxadiazol
2-Chlormethyl-5-methylthio-1,3,4-oxadiazol
2-Chlormethyl-5-allylthio-1,3,4-oxadiazol
2-Chlormethyl-5-(2-fluorbenzylthio)-1,3,4-oxadiazol
2-(1-Chloräthyl)-5-methyl-1,3,4-oxadiazol
2-(1-Chloräthyl)-5-äthyl-1,3,4-oxadiazol
2-Chlormethyl-5-methyl-1,3,4-thiadiazol
2-Chlormethyl-5-äthyl-1,3,4-thiadiazol
2-Chlormethyl-5-isopropyl-1,3,4-thiadiazol
2-Chlormethyl-5-trifluormethyl-1,3,4-thiadiazol
2-Chlormethyl-5-trichlormethyl-1,3,4-thiadiazol
2-Chlormethyl-5-allyl-1,3,4-thiadiazol
2-Chlormethyl-5-cyclohexyl-1,3,4-thiadiazol
2-Chlormethyl-5-benzyl-1,3,4-thiadiazol
2-Chlormethyl-5-methoxy-1,3,4-thiadiazol
2-Chlormethyl-5-allyloxy-1,3,4-thiadiazol
2-Chlormethyl-5-(2-fluorbenzyloxy)-1,3,4-thiadiazol
2-Chlormethyl-5-methylthio-1,3,4-thiadiazol
2-Chlormethyl-5-allylthio-1,3,4-thiadiazol
2-Chlormethyl-5-(2-fluorbenzylthio)-1,3,4-thiadiazol
2-(1-Chloräthyl)-5-methyl-1,3,4-thiadiazol
2-(1-Chloräthyl)-5-äthyl-1,3,4-thiadiazol
2-Chlormethyl-5-methyl-1,3,4-triazol
2-Chlormethyl-5-äthyl-1,3,4-triazol
2-Chlormethyl-5-isopropyl-1,3,4-triazol
2-Chlormethyl-5-trifluormethyl-1,3,4-triazol
2-Chlormethyl-5-trichlormethyl-1,3,4-triazol
2-Chlormethyl-5-allyl-1,3,4-triazol
2-Chlormethyl-5-cyclohexyl-1,3,4-triazol
2-Chlormethyl-5-benzyl-1,3,4-triazol
2-Chlormethyl-5-methoxy-1,3,4-triazol
2-Chlormethyl-5-allyloxy-1,3,4-triazol
2-Chlormethyl-5-(2-fluorbenzyloxy)-1,3,4-triazol

2-Chlormethyl-5-methylthio-1,3,4-triazol
2-Chlormethyl-5-allylthio-1,3,4-triazol
2-Chlormethyl-5-(2-fluorbenzylthio)-1,3,4-triazol
2-(1-Chloräthyl)-5-methyl-1,3,4-triazol
2-(1-Chloräthyl)-5-äthyl-1,3,4-triazol
2-Chlormethyl-1,5-dimethyl-1,3,4-triazol
2-Chlormethyl-5-äthyl-1-methyl-1,3,4-triazol
2-Chlormethyl-5-isopropyl-1-methyl-1,3,4-triazol
2-Chlormethyl-1-methyl-5-trifluormethyl-1,3,4-triazol
2-Chlormethyl-1-methyl-5-trichlormethyl-1,3,4-triazol
2-Chlormethyl-5-allyl-1-methyl-1,3,4-triazol
2-Chlormethyl-5-cyclohexyl-1-methyl-1,3,4-triazol
2-Chlormethyl-5-benzyl-1-methyl-1,3,4-triazol
2-Chlormethyl-5-methoxy-1-methyl-1,3,4-triazol
2-Chlormethyl-5-allyloxy-1-methyl-1,3,4-triazol
2-Chlormethyl-5-(2-fluorbenzyloxy)-1-methyl-1,3,4-triazol
2-Chlormethyl-5-methylthio-1-methyl-1,3,4-triazol
2-Chlormethyl-5-allylthio-1-methyl-1,3,4-triazol
2-Chlormethyl-5-(2-fluorbenzylthio)-1-methyl-1,3,4-triazol
2-(1-Chloräthyl)-1,5-dimethyl-1,3,4-triazol
2-(1-Chloräthyl)-5-äthyl-1-methyl-1,3,4-triazol

Die Ausgangsstoffe der Formel (VI) sind noch nicht bekannt. Sie lassen sich jedoch nach bekannten Verfahren herstellen, indem man bekannte Ester (vergleiche u. a. DE-OS 2 350 944 und 2 513 730) der Formel

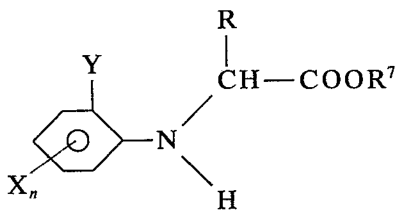
(XVII)

in welcher
R, X, Y und n die oben angegebene Bedeutung haben und
$R^7$ für Methyl oder Äthyl steht,

mit Hydrazinhydrat vorzugsweise in Gegenwart eines organischen Lösungsmittels, wie beispielsweise Äthanol, Dioxan oder Dimethylformamid, bei Temperaturen zwischen 20 und 120°C umsetzt (vergleiche auch Herstellungsbeispiele).

Als Beispiele seien genannt:

2,6-Diäthylanilino-essigsäure-hydrazid
2,6-Dimethylanilino-essigsäure-hydrazid
2,4,6-Trimethylanilino-essigsäure-hydrazid
2-Äthyl-6-methylanilino-essigsäure-hydrazid
α-(2,6-Diäthylanilino)-propionsäure-hydrazid
α-(2,6-Dimethylanilino)-propionsäure-hydrazid
α-(2,4,6-Trimethylanilino)-propionsäure-hydrazid
α-(2-Äthyl-6-methylanilino)-propionsäure-hydrazid

Die Ausgangsstoffe der Formeln (VII), (X), (XIa), (XIb), (XIII) und (XIV) sind allgemein bekannte Verbindungen der organischen Chemie.

In den Verbindungen der Formeln (XIa) und (XIb) steht $R^5$ vorzugsweise für geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen. $R^5$ steht ferner vorzugsweise für Phenyl, wobei dieser Phenylrest substituiert sein kann durch Halogen, Alkyl mit 1 bis 4 Kohlenstoffatomen, Alkoxy mit 1 bis 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^5$ steht ferner vorzugsweise für Benzyl, wobei dieser

Benzylrest im Phenylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei.

In den Verbindungen der Formel (XIII) steht $R^6$ vorzugsweise für Alkyl mit 1 bis 4 Kohlenstoffatomen, Halogenalkyl mit 1 bis 4 Kohlenstoffatomen und 1 bis 5 Halogenatomen oder für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Nitro substituiertes Phenyl.

In den Iminoäthern der Formel (XIV) steht $R^5$ vorzugsweise für diejenigen Reste, die bereits im Zusammenhang mit der Beschreibung der Verbindungen der Formel (XIa) und (XIb) vorzugsweise für $R^5$ genannt wurden.

Die Ausgangsstoffe der Formel (XV) bzw. deren Herstellung sind ebenfalls bekannt (vergleiche Elderfield, Heterocyclic Compounds, Vol. 7 [1961] und Advances in Heterocyclic Chemistry, Vol. 9 [1968]).

Die außerdem für die erfindungsgemäße Umsetzung als Ausgangsstoffe zu verwendenden Halogenessigsäurechloride bzw. -anhydride sind durch die Formeln (IIIa) und (IIIb) allgemein definiert. In diesen Formeln steht Z vorzugsweise für Chlor, Brom und Jod.

Die Halogenessigsäurechloride und -anhydride der Formeln (IIIa) und (IIIb) sind allgemein bekannte Verbindungen der organischen Chemie. Als Beispiele seien genannt: Chloracetylchlorid, Bromacetylchlorid, Jodacetylchlorid und die entsprechenden Anhydride.

Als Verdünnungsmittel kommen für die erfindungsgemäße Umsetzung vorzugsweise inerte organische Lösungsmittel infrage. Hierzu gehören vorzugsweise Ketone, wie Diäthylketon, insbesondere Aceton und Methyläthylketon; Nitrile, wie Propionitril, insbesondere Acetonitril; Äther, wie Tetrahydrofuran oder Dioxan; aliphatische und aromatische Kohlenwasserstoffe, wie Petroläther, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren kann gegebenenfalls in Gegenwart von Säurebindern (Chlorwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triäthylamin, oder wie Pyridin; ferner anorganische Basen, wie beispielsweise Alkalihydroxide und Alkalicarbonate.

Die Reaktionstemperaturen können bei der Durchführung des erfindungsgemäßen Verfahrens in einem größeren Bereich variiert werden. Im allgemeinen arbeitet man zwischen 0 und 120°C, vorzugsweise zwischen 20 und 100°C.

Bei der Durchführung des erfindungsgemäßen Verfahrens setzt man vorzugsweise auf 1 Mol der Verbindung der Formel (II) 1 bis 1,5 Mol Halogenacetylierungsmittel und 1 bis 1,5 Mol Säurebinder ein. Die Isolierung der Verbindungen der Formel (I) erfolgt in üblicher Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Spenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeichnen sich neben der guten Totalherbizid-Wirkung vor allem durch ihre selektiven Einsatzmöglichkeiten in wichtigen Kulturpflanzen, wie Baumwolle, Rüben, Raps, Getreide, Mais und Sojabohnen. Sie eignen sich vornehmlich zur pre-emergence-Anwendung, aber auch bei post-emergence-Anwendung zeigen sie Wirkung. Zur Verbesserung der Wirkung sind Kombinationen mit Metribuzin, Metamitron, Methabenzthiazuron, Diuron, Fluometuron u. a. möglich.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Aerosole, Suspensions-Emulsionskonzentrate, Saatgutpuder, Wirkstoff-imprägnierte Natur- und synthetische Stoffe und Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser, als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z. B. Alkylaryl-polyglykol-äther, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen. Wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z. B. durch Gießen, Spritzen, Sprühen, Stäuben, Streuen, Trockenbeizen, Feuchtbeizen, Naßbeizen, Schlämmbeizen oder Inkrustieren.

Die erfindungsgemäßen Wirkstoffe können sowohl nach als auch insbesondere vor Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 20 kg Wirkstoff pro ha, vorzugsweise zwischen 0,25 und 10 kg/ha.

In dem nachfolgenden Beispiel wird die nachstehend angegebene Verbindung als Vergleichssubstanz eingesetzt:

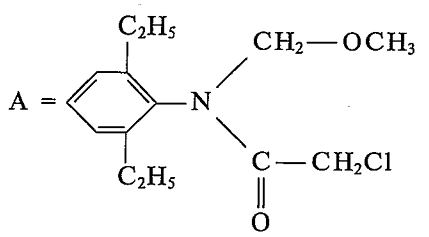

(2,6-Diäthyl-N-methyloxymethyl-chloracetanilid).

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Die erfindungsgemäßen Wirkstoffe (1), (3), (4) und (5) zeigen in diesem Test eine bessere selektive herbizide Wirksamkeit als die aus dem Stand der Technik bekannte Substanz (A).

Herstellungsbeispiele

Beispiel 1

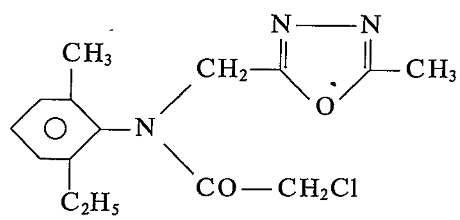

16,3 g (0,07 Mol) 2-Äthyl-6-methyl-N-[(2-methyl-1,3,4-oxadiazol-5-yl)-methyl]-anilin und 6 g (0,076 Mol) wasserfreies Pyridin werden in 100 ml absolutem Tetrahydrofuran unter Rühren zum Sieden erhitzt und tropfenweise mit einer Lösung von 8 g (0,07 Mol) Chloracetylchlorid in 20 ml Tetrahydrofuran versetzt. Nach beendetem Zutropfen läßt man 10 Minuten nachrühren, engt durch Abdestillieren des Lösungsmittels ein und verrührt den Rückstand mit 150 ml Wasser. Das auskristallisierende Reaktionsprodukt wird abgesaugt, mit Wasser gewaschen und getrocknet. Man erhält 18,7 g (87% der Theorie) beige-farbene Kristalle von 2-Äthyl-6-methyl-N-[(2-methyl-1,3,4-oxadiazol-5-yl)-methyl]-chloracetanilid vom Schmelzpunkt 67 – 70° C.

Herstellung des Ausgangsproduktes

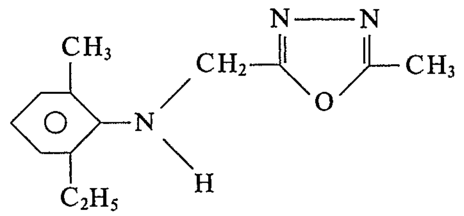

Eine Mischung aus 101,2 g (0,76 Mol) 2-Äthyl-6-methyl-anilin, 40 g (0,3 Mol) 5-Chlormethyl-1,3,4-oxadiazol, 41,4 g (0,3 Mol) gepulvertes Kaliumcarbonat und 76 ml Dimethylformamid wird 5 Stunden unter Rühren auf 100°C erhitzt. Danach wird die Reaktionsmischung filtriert, das Filtrat mit Methylenchlorid verdünnt und mehrmals mit Wasser gewaschen. Die Methylenchloridphase wird über Natriumsulfat getrocknet und im Vakuum durch Abdestillieren des Lösungsmittels eingeengt. Der Rückstand wird im Vakuum destilliert. Man erhält 46,8 g (67,5% der Theorie) gelbliches Öl von 2-Äthyl-6-methyl-N-[(2-methyl-1,3,4-oxadiazol-5-yl)-methyl]-anilin vom Siedepunkt 140 – 142° C/0,1 mm mit einer 94%igen Reinheit (Gaschromatografisch bestimmt).

Beispiel 2

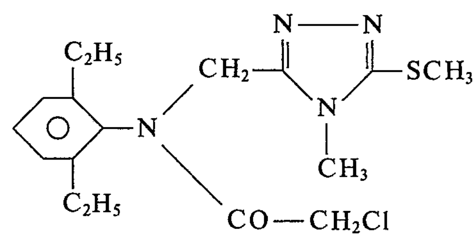

5 g (0,017 Mol) 2,6-Diäthyl-N-[(1-methyl-2-methylthio-1,3,4-triazol-5-yl)-methyl]-anilin und 1,6 g (0,02 Mol) Pyridin werden in 100 ml absolutem Tetrahydrofuran gerührt und bei Raumtemperatur tropfenweise mit 2,3 g (0,02 Mol) Chloracetylchlorid versetzt, wobei die Temperatur auf ca. 30°C ansteigt. Man läßt 2 Stunden rühren, engt teilweise durch Abdestillieren des Lösungsmittels ein und versetzt mit Wasser. Das auskristallisierende Produkt wird abgesaugt, getrocknet und aus Diisopropyläther/Essigester umkristallisiert. Man erhält 5 g (80% der Theorie) 2,6-Diäthyl-N-[(1-methyl-2-methylthio-1,3,4-triazol-5-yl)-methyl]-chloracetanilid vom Schmelzpunkt 121 – 123° C.

Herstellung der Vorstufen

a) 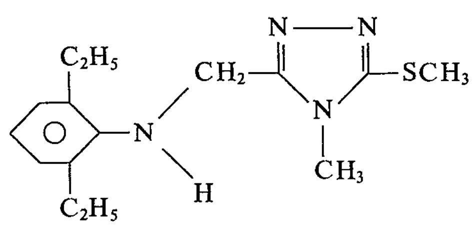

13,9 g (0,05 Mol) 2,6-Diäthyl-N-[(1-methyl-2-thiono-1,3,4-triazol-5-yl)-methyl]-anilin werden bei Raumtemperatur in einem Zweiphasengemisch aus 150 ml Toluol und 40 ml 50%iger Natronlauge unter Zusatz von 1,5 g Triäthyl-benzyl-ammoniumchlorid (TEBA) als Katalysator schnell gerührt und tropfenweise mit 6,3 g (0,05 Mol) Dimethylsulfat versetzt, wobei die Temperatur auf ca. 35°C ansteigt. Man läßt 5 Stunden rühren, trennt die Toluolphase ab, wäscht sie mehrmals mit Wasser, trocknet über Natriumsulfat und engt durch Abdestillieren des Lösungsmittels ein. Das zurückbleibende Öl wird durch Zusatz von Petroläther zur Kristallisation gebracht. Man erhält nach Umkristallisation aus Petroläther 6,7 g (40% der Theorie) 2,6-Diäthyl-N-[1-methyl-2-methylthio-1,3,4-triazol-5-yl)-methyl]-anilin vom Schmelzpunkt 65 – 67° C.

b) 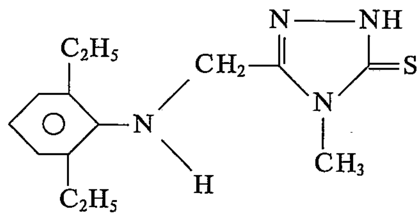

29,6 g (0,1 Mol) 1-Methyl-4-[(2,6-diäthyl-anilino)-acetyl]-thiosemicarbazid werden in 150 ml Äthanol suspendiert und nach Zugabe von 7 g Kaliumhydroxid in 20 ml Wasser 1 Stunde unter Rückfluß erhitzt. Danach wird der größte Teil des Lösungsmittels abdestilliert und der Rückstand mit 250 ml Wasser versetzt. Nach dem Ansäuern mit Eisessig auf pH 5 wird der entstehende Niederschlag abgesaugt und gründlich mit Wasser gewaschen. Nach dem Trocknen erhält man 27 g (97% der Theorie) 2,6-Diäthyl-N-[(1-methyl-2-thiono-1,3,4-triazol-5-yl)-methyl]-anilin vom Schmelzpunkt 117 – 121° C.

c) 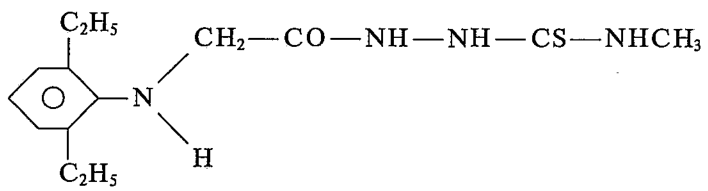

44,2 g (0,2 Mol) 2,6-Diäthyl-anilino-essigsäure-hydrazid und 14,8 g (0,2 Mol) Methylsenföl werden in 250 ml Äthanol gelöst und eine Stunde auf Rückflußtemperatur erhitzt. Nach dem anschließenden Abkühlen auf Raumtemperatur wird der entstandene Niederschlag abgesaugt und zweimal mit je 50 ml Äthanol nachgewaschen. Nach dem Trocknen erhält man 46 g (78% der Theorie) an 1-Methyl-4[2,6-diäthyl-anilino)-acetyl]-thiosemicarbazid in Form einer farblosen kristallinen Substanz von Schmelzpunkt 166° C.

d) 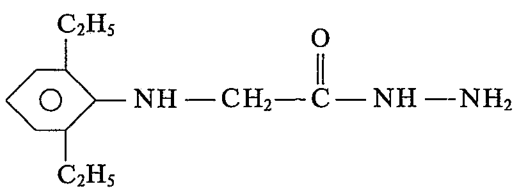

58,7 g (0,25 Mol) 2,6-Diäthyl-anilino-essigsäureäthylester und 25 g Hydrazinhydrat werden in 200 ml Äthanol 24 Stunden stehen gelassen. Danach wird durch Abdestillieren des Lösungsmittels eingeengt und der Rückstand mit Wasser ausgerührt. Nach dem Trocknen erhält man 50,5 g (91% der Theorie) farblose Kristalle von 2,6-Diäthyl-anilino-essigsäurehydrazid vom Schmelzpunkt 71 – 73° C.

In entsprechender Weise werden diejenigen Verbindungen erhalten, die in der Tabelle 1 formelmäßig aufgeführt sind.

Tabelle 1

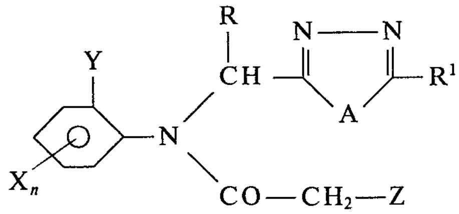

(I)

| Bsp. Nr. | R | $R^1$ | Y | $X_n$ | A | Z | Schmelz- punkt (°C) |
|---|---|---|---|---|---|---|---|
| 3 | H | $CH_3$ | $C_2H_5$ | 6-$C_2H_5$ | O | Cl | 79–82 |
| 4 | H | $CH_3$ | $CH_3$ | 6-$CH_3$ | O | Cl | 91–93 |
| 5 | H | $CH_3$ | $C(CH_3)_3$ | — | O | Cl | 102–104 |
| 6 | H | $—S—CH_2—CH=CH_2$ | $C_2H_5$ | 6-$C_2H_5$ | 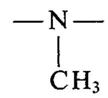 | Cl | 67–70 |
| 7 | H | 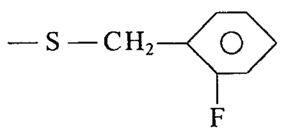 | $CH_3$ | 6-$C_3H_5$ | 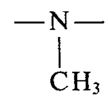 | Cl | 115–120 |
| 8 | H | $C_2H_5$ | $CH_3$ | 6-$C_2H_5$ | O | Cl | 57–59 |
| 9 | H | $C_2H_5$ | $C_2H_5$ | 6-$C_2H_5$ | O | Cl | 43–47 |
| 10 | H | i-$C_3H_7$ | $CH_3$ | 6-$C_2H_5$ | O | Cl | zähfl. Öl |
| 11 | H | $CH_3$ | $CH_3$ | 3-$CH_3$ | 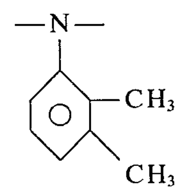 | Cl | glasartig erstarrt |
| 12 | H | $CH_3$ | $C_2H_5$ | 6-$C_2H_5$ | O | Br | 80 |
| 13 | H | $CH_3$ | $CH_3$ | 6-$C_2H_5$ | O | Br | 92–94 |
| 14 | H | $CH_3$ | i-$C_3H_7$ | 6-i-$C_3H_7$ | O | Cl | 135–137 |

Nach einem oder mehreren der in der Anmeldung beschriebenen Verfahren werden die in der nachstehenden Tabelle formelmäßig aufgeführten Ausgangsprodukte erhalten.

Tabelle 2

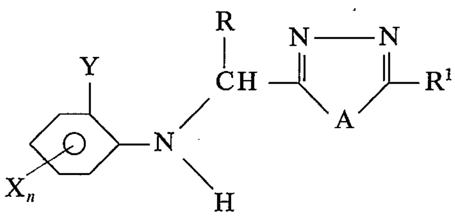

(I)

| Beispiel Nr. | R | $R^1$ | Y | $X_n$ | A | Schmelzpunkt [°C] bzw. Brechungsindex |
|---|---|---|---|---|---|---|
| II-1 | H | $CH_3$ | $C_2H_5$ | 6-$C_2H_5$ | O | $n_D^{22}$ = 1,540 |
| II-2 | H | $CH_3$ | $CH_3$ | 6-$C_2H_5$ | O | $n_D^{22}$ = 1,547 |
| II-3 | H | $CH_3$ | $CH_3$ | 6-$CH_3$ | O | $n_D^{22}$ = 1,552 |
| II-4 | H | $CH_3$ | $-(CH_3)_3$ | — | O | 52–55 |
| II-5 | H | $CH_3$ | i-$C_3H_7$ | 6-i-$C_3H_7$ | O | 96–99 |
| II-6 | H | $C_2H_5$ | $C_2H_5$ | 6-$C_2H_5$ | O | $n_D^{22}$ = 1,534 |
| II-7 | H | $C_2H_5$ | $CH_3$ | 6-$C_2H_5$ | O | $n_D^{21}$ = 1,542 |
| II-8 | H | i-$C_3H_7$ | $CH_3$ | 6-$C_2H_5$ | O | $n_D^{21}$ = 1,531 |
| II-9 | H | $SCH_3$ | $C_2H_5$ | 6-$C_2H_5$ | 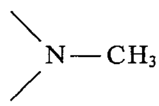 | 64–57 |
| II-10 | H | $S-CH_2-CH=CH_2$ | $C_2H_5$ | 6-$C_2H_5$ | 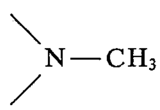 | $n_D^{21}$ = 1,577 |
| II-11 | H | 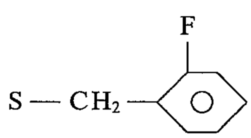 | $CH_3$ | 6-$C_2H_5$ | 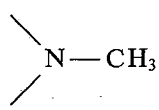 | zähes Öl |
| II-12 | H | $CH_3$ | $CH_3$ | 3-$CH_3$ | 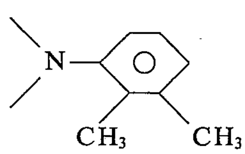 | 142–143 |

**Patentansprüche**

1. N-Azolylalkyl-halogenacetanilide der Formel

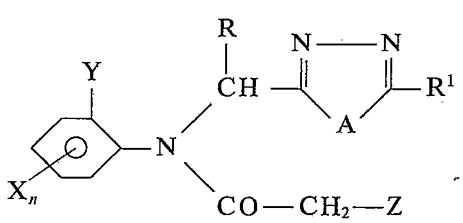

(I)

in welcher
A für Sauerstoff, Schwefel oder die Gruppierung >$NR^2$ steht,
R für Wasserstoff oder Methyl steht,

$R^1$ für Wasserstoff, Alkyl mit 1 bis 4 Kohlenstoffatomen, Halogenalkyl mit bis zu 3 Kohlenstoffatomen und bis zu 5 gleichen oder verschiedenen Halogenatomen, Alkenyl mit 2 bis 4 Kohlenstoffatomen, Alkinyl mit 2 bis 4 Kohlenstoffatomen, Cycloalkyl mit 5 bis 7 Kohlenstoffatomen, Halogen, gegebenenfalls durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen substituiertes Aryl mit 6 bis 10 Kohlenstoffatomen sowie für Aralkyl mit 6 bis 10 Kohlenstoffatomen im Arylteil und 1 bis 4 Kohlenstoffatomen im Alkylteil steht, wobei der Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, oder $R^1$ für die Gruppierungen $-OR^3$, $-SR^3$ und $-NR^2R^3$ steht,

$R^2$ für Wasserstoff, Alkyl mit 1 bis 4 Kohlenstoffatomen oder für gegebenenfalls durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen substituiertes Aryl mit 6 bis 10 Kohlenstoffatomen steht,

$R^3$ für Wasserstoff, Alkyl mit 1 bis 4 Kohlenstoffatomen, Halogenalkyl mit bis zu 3 Kohlenstoffatomen und bis zu 5 gleichen oder verschiedenen Halogenatomen, Alkenyl mit 2 bis 4 Kohlenstoffatomen, Alkinyl mit 2 bis 4 Kohlenstoffatomen, Cycloalkyl mit 5 bis 7 Kohlenstoffatomen oder für Aralkyl mit 6 bis 10 Kohlenstoffatomen im Arylteil und 1 bis 4 Kohlenstoffatomen im Alkylteil steht, wobei der Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen,

X für Alkyl mit 1 bis 4 Kohlenstoffatomen steht,

Y für Alkyl mit 1 bis 4 Kohlenstoffatomen oder Halogen steht,

Z für Halogen steht und

n für die Zahlen 0, 1 oder 2 steht.

2. Verfahren zur Herstellung von N-Azolylalkyl-halogenacetaniliden, dadurch gekennzeichnet, daß man N-Azolylalkylaniline der Formel

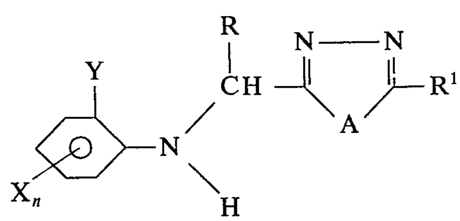

(II)

in welcher

A, R, $R^1$, X, Y und n die oben angegebene Bedeutung haben,

mit Halogenessigsäurechloriden bzw. -anhydriden der Formeln

$$Z-CH_2-CO-Cl \quad (IIIa)$$

bzw.

$$(Z-CH_2-CO)_2O \quad (IIIb)$$

in welchen

Z die oben angegebene Bedeutung hat,

in Gegenwart eines Verdünnungsmittels und gegebenenfalls in Gegenwart eines Säurebindemittels umsetzt.

3. Herbizide Mittel, gekennzeichnet durch einen Gehalt an mindestens einem N-Azolylalkylhalogenacetanilid gemäß Anspruch 1.

4. Verwendung von N-Azolylalkyl-halogenacetaniliden gemäß Anspruch 1 zur Bekämpfung von Unkräutern.

**Claims**

1. N-Azolylalkyl-halogenoacetanilides of the formula

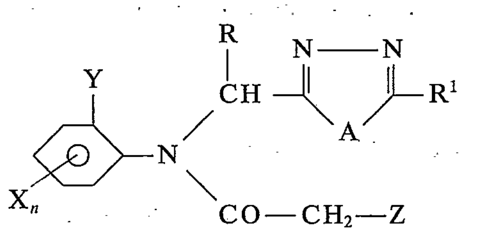

(I)

in which

A represents oxygen, sulphur or the grouping $>NR^2$,

R represents hydrogen or methyl,

$R^1$ represents hydrogen, alkyl with 1 to 4 carbon atoms, halogenoalkyl with up to 3 carbon atoms and up to 5 identical or different halogen atoms, alkenyl with 2 to 4 carbon atoms, alkinyl with 2 to 4 carbon atoms, cycloalkyl with 5 to 7 carbon atoms, halogen, aryl having 6 to 10 carbon atoms and being optionally substituted by halogen, alkyl with 1 to 4 carbon atoms, alkoxy with 1 or 2 carbon atoms, alkylthio with 1 or 2 carbon atoms, cyano, nitro and/or halogenoalkyl with up to 2 carbon atoms and up to 5 identical or different halogen atoms; as well as aralkyl with 6 to 10 carbon atoms in the aryl part and 1 to 4 carbon atoms in the alkyl part, it being possible for the aryl part to be substituted by halogen, alkyl with 1 to 4 carbon atoms, alkoxy with 1 or 2 carbon atoms, alkylthio with 1 or 2 carbon atoms, cyano, nitro and/or halogenoalkyl with up to 2 carbon atoms and up to 5 identical or different halogen atoms, or $R^1$ represents the groupings $-OR^3$, $-SR^3$ and $-NR^2R^3$,

$R^2$ represents hydrogen, alkyl with 1 to 4 carbon atoms, or aryl having 6 to 10 carbon atoms and optionally being substituted by halogen, alkyl with 1 to 4 carbon atoms, alkoxy with 1 or 2 carbon atoms, alkylthio with 1 or 2 carbon atoms, cyano, nitro and/or halogenoalkyl with up to 2 carbon atoms and up to 5 identical or different halogen atoms,

$R^3$ represents hydrogen, alkyl with 1 to 4 carbon atoms, halogenoalkyl with up to 3 carbon atoms and up to 5 indentical or different halogen atoms, alkenyl with 2 to 4 carbon atoms, alkinyl with 2 to 4 carbon atoms, cycloalkyl with 5 to 7 carbon atoms or aralkyl with 6 to 10 carbon atoms in the aryl part and 1 to 4 carbon atoms in the alkyl part, it beeing possible for the aryl part to be substituted by halogen, alkyl with 1 to 4 carbon atoms, alkoxy with 1 or 2 carbon atoms, alkylthio with 1 or 2 carbon atoms, cyano, nitro and/or halogenoalkyl with up to 2 carbonatoms and up to 5 identical or different halogen atoms,

X represents alkyl with 1 to 4 carbon atoms,

Y represents alkyl with 1 to 4 carbon atoms or halogen,

Z represents halogen and

n represents the numbers 0, 1 or 2.

2. Process for the preparation of N-azolylalkyl-halogenoacetanilides, characterised in that N-azolylalkyl-anilines of the formula

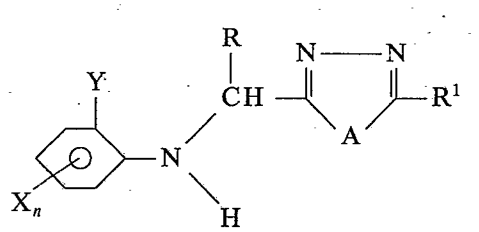

(II)

in which

A, R, $R^1$, X, Y and n have the meaning indicated above,

are reacted with halogenoacetic acid chlorides or anhydrides of the formulae

$$Z-CH_2-CO-Cl \quad \text{(IIIa)}$$

or

$$(Z-CH_2-CO)_2O \quad \text{(IIIb)}$$

in which

Z has the meaning indicated above,

in the presence of a diluent and optionally in the presence of an acid-binding agent.

3. Herbicidal agents, characterised in that they contain at least one N-azolylalkylhalogenoacetanilide according to Claim 1.

4. Use of N-azolylalkyl-halogenoacetanilides according to Claim 1 for combating weeds.

**Revendications**

1. N-azolylalkylhalogénoacétanilides de formule

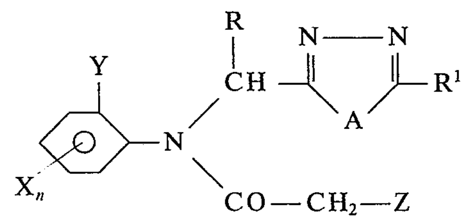

(I)

dans laquelle:

A représente l'oxygène, le soufre ou le groupement $>NR^2$,

R représente l'hydrogène ou un groupe méthyle,

$R^1$ représente l'hydrogène, un groupe alkyle en $C_1-C_4$, halogénoalkyle jusqu'en $C_3$ et contenant jusqu'à 5 atomes d'halogènes identiques ou différents, alcényle en $C_2-C_4$, alcynyle en $C_2-C_4$, cycloalkyle en $C_5-C_7$, un halogène, un groupe aryle en $C_6-C_{10}$ éventuellement substitué par un halogène, un groupe alkyle en $C_1-C_4$, alcoxy en $C_1$ ou $C_2$, alkylthio en $C_1$ ou $C_2$, cyano, nitro et/ou halogénoalkyle jusqu'en $C_2$ et contenant jusqu'à 5 atomes d'halogènes identiques ou différents ainsi qu'un groupe (aryl en $C_6-C_{10}$)alkyle en $C_1-C_4$ dans lequel le reste aryle peut être substitué par un halogène, un groupe alkyle en $C_1-C_4$, alcoxy en $C_1-C_2$, alkylthio en $C_1$ ou $C_2$, cyano, nitro et/ou halogénoalkyle jusqu'en $C_2$ et dontenant jusqu'à 5 atomes d'halogènes identiques ou différents, ou bien $R_1$ représente les groupes $-OR^3$, $-SR^3$ et $-NR^2R^3$,

$R^2$ représente l'hydrogène, un groupe alkyle en $C_1-C_4$ ou un groupe aryle en $C_6-C_{10}$ éventuellement substitué par un halogène, un groupe alkyle en $C_1-C_4$, alcoxy en $C_1$ ou $C_2$, alkylthio en $C_1$ ou $C_2$, cyano, nitro et/ou halogénoalkyle jusqu'en $C_2$ et contenant jusqu'à 5 atomes d'halogènes identiques ou différents,

$R^3$ représente l'hydrogène, un groupe alkyle en $C_1-C_4$, halogénoalkyle jusqu'en $C_3$ et contenant jusqu'à 5 atomes d'halogènes identiques ou différents, alcényle en $C_2-C_4$, alcynyle en $C_2-C_4$, cycloalkyle en $C_5-C_7$ ou (aryl en $C_6-C_{10}$)alkyle en $C_1-C_4$, le reste aryle pouvant être substitué par un halogène, un groupe alkyle en $C_1-C_4$, alcoxy en $C_1$ ou $C_2$, alkylthio en $C_1$ ou $C_2$, cyano, nitro et/ou halogénoalkyle jusqu'en $C_2$ et contenant jusqu'à 5 atomes d'halogènes identiques ou différents,

X représente un groupe alkyle en $C_1-C_4$,

Y représente un groupe alkyle en $C_1-C_4$ ou un halogène,

Z représente un halogène, et

n représente les nombres 0, 1 ou 2.

2. Procédé pour la préparation de N-azolylalkylhalogénoacétanilides, caractérisé en ce qu'on fait réagir des N-azolylalkylanilines de formule

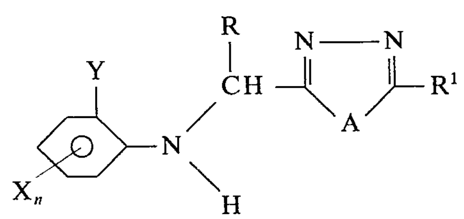

(II)

dans laquelle A, R, $R^1$, X, Y et n ont la signification indiquée cidessus avec des chlorures ou anhydrides d'acides halogénoacétiques de formule

$$Z-CH_2-CO-Cl \quad \text{(IIIa)}$$

ou

$$(Z-CH_2-CO)_2O \quad \text{(IIIb)}$$

dans lesquelles Z a la signification indiquée ci-dessus, en présence d'un agent diluant et, éventuellement, en présence d'un accepteur d'acide.

3. Agents herbicides caractérisés en ce qu'ils contiennent au moins un N-azolylalkylhalogénoacétanilide selon la revendication 1.

4. Utilisation des N-azolylalkylhalogénoacétanilides selon la revendication 1 pour la lutte contre las mavaises herbes.